# EUROPEAN PATENT SPECIFICATION

(11) **EP 2 689 246 B1**
(45) Date of publication and mention of the grant of the patent: **18.10.2017**
(21) Application number: 12762965.7
(22) Date of filing: 26.03.2012
(51) Int. Cl.: G01N 33/48, G01N 33/68, C12Q 1/68, G01N 33/564

(54) **METHODS OF DIAGNOSING ULCERATIVE COLITIS AND CROHN'S DISEASE**
VERFAHREN ZUR DIAGNOSE VON COLITIS ULCEROSA UND MORBUS CROHN
MÉTHODES DE DIAGNOSTIC DE LA COLITE ULCÉREUSE ET DE LA MALADIE DE CROHN

(30) Priority: 25.03.2011 US 201161467872 P; 25.03.2011 US 201161467893 P
(43) Date of publication of application: 29.01.2014
(73) Proprietor: Cedars-Sinai Medical Center, Los Angeles, CA 90048 (US)
(72) Inventor: TAYLOR, Kent D., Torrance, California 90501 (US); McGOVERN, Dermot P., Los Angeles California 90049 (US); ROTTER, Jerome I., Los Angeles California 90064 (US); TARGAN, Stephan R., Santa Monica California 90402 (US); HARITUNIANS, Talin, Los Angeles California 90048 (US)
(74) Representative: HGF Limited
(86) International application number: PCT/US2012/030611
(87) International publication number: WO 2012/135142

(56) References cited:
- WO-A1-2010/118210
- US-A1- 2010 254 971
- US-A1- 2010 254 971
- US-A1- 2010 284 999
- US-A1- 2010 284 999
- US-A1- 2011 033 486
- US-A1- 2011 033 486
- US-A1- 2011 045 476
- DATABASE UNIPROT [Online] 08 March 2011 MORINAGA ET AL., XP002405745 Database accession no. P02771
- MORINAGA ET AL.: 'Primary structures of human .alpha.-fetoprotein and it mRNA' PNAS vol. 80, August 1983, USA, pages 4604 - 4608, XP001062510

## Description

### FIELD OF INVENTION

The invention relates to the field of genetics and medicine. More specifically, the invention relates to methods of diagnosing and prognosing inflammatory bowel disease including ulcerative colitis and Crohn's disease.

### BACKGROUND

The following description includes information that may be useful in understanding the present invention. It is not an admission that any of the information provided herein is prior art or relevant to the presently claimed invention, or that any publication specifically or implicitly referenced is prior art.

Crohn's disease (CD) and ulcerative colitis (UC), the two common forms of idiopathic inflammatory bowel disease (IBD), are chronic, relapsing inflammatory disorders of the gastrointestinal tract. Each has a peak age of onset in the second to fourth decades of life and prevalences in European ancestry populations that average approximately 100-150 per 100,000 (D.K. Podolsky, N Engl J Med 347, 417 (2002); E.V. Loftus, Jr., Gastroenterology 126, 1504 (2004)). Although the precise etiology of IBD remains to be elucidated, a widely accepted hypothesis is that ubiquitous, commensal intestinal bacteria trigger an inappropriate, overactive, and ongoing mucosal immune response that mediates intestinal tissue damage in genetically susceptible individuals (D.K. Podolsky, N Engl J Med 347, 417 (2002)). Genetic factors play an important role in IBD pathogenesis, as evidenced by the increased rates of IBD in Ashkenazi Jews, familial aggregation of IBD, and increased concordance for IBD in monozygotic compared to dizygotic twin pairs (S. Vermeire, P. Rutgeerts, Genes Immun 6, 637 (2005)). Moreover, genetic analyses have linked IBD to specific genetic variants, especially CARD15 variants on chromosome 16q12 and the IBD5 haplotype (spanning the organic cation transporters, SLC22A4 and SLC22A5, and other genes) on chromosome 5q31 (S. Vermeire, P. Rutgeerts, Genes Immun 6, 637 (2005); J.P. Hugot et al., Nature 411, 599 (2001); Y. Ogura et al., Nature 411, 603 (2001); J.D. Rioux et al., Nat Genet 29, 223 (2001); V.D. Peltekova et al., Nat Genet 36, 471 (2004)). CD and UC are thought to be related disorders that share some genetic susceptibility loci but differ at others.

US2010/284999 discloses a method of prognosing IBD in an individual by assaying a sample obtained from the individual to determine the presence or absence of certain risk factors on Chromosome 4 and assaying for the absence or presence of serological marker ANCA. WO 2010/118210 concerns methods of prognosing Crohn's disease by examining genetic risk variants at a number of genetic loci. US 2010/254971 discloses methods of prognosing IBD or Crohn's disease by measuring levels of antibodies to glycans in a biological sample. US2011/033486 refers to the analysis of gene expression profiling for assessing the pathogenesis of IBD with the AFP locus cited as one of the affected loci in the pathogenesis.

### SUMMARY OF THE INVENTION

The present invention concerns a method of prognosing inflammatory bowel disease (IBD) in an individual, comprising:
assaying a sample obtained from the individual to determine the presence or absence of one or more risk variants selected from rs10001225(SEQ ID No. 2) rs4694164(SEQ ID No. 3) and rs7668327(SEQ ID No. 4),
assaying the sample to determine the presence or absence of serological marker ANCA; and
prognosing an aggressive form of inflammatory bowel disease in the individual based on the presence of one or more of the risk variants and the presence of serological marker ANCA.

Described herein are methods, including methods of prognosing inflammatory bowel disease (IBD) in an individual, comprising obtaining a sample, assaying the sample to determine the presence or absence of one or more risk variants at Chromosome 4, assaying the sample to determine the presence or absence of serological marker ANCA, prognosing an aggressive form of inflammatory bowel disease in the individual based on the presence of one or more risk variants at Chromosome 4 and the presence of serological marker ANCA. In another embodiment, the aggressive form of inflammatory bowel disease is characterized by an aggressive form of ulcerative colitis. In another embodiment, the presence of serological marker ANCA comprises a high level of serological marker ANCA as compared to a healthy subject. In another embodiment, the absence of serological marker ANCA is indicative of inflammatory bowel disease with Crohn's like conditions.

Other features and advantages of the invention will become apparent from the following detailed description, taken in conjunction with the accompanying drawings, which illustrate, by way of example, various embodiments of the invention.

### BRIEF DESCRIPTION OF THE FIGURES

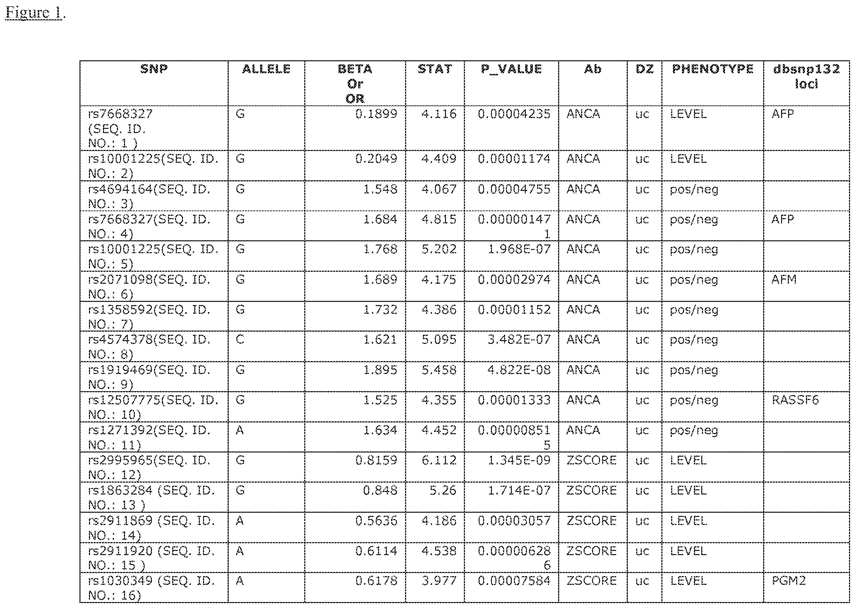

Figure 1 depicts two Chr.4 loci associated with the phenotypic difference of UC patients, one for UC severity as typified by ANCA level, the other for the expression of antibodies more characteristic of CD. If a listed SNP allele has an OR>1, it means that this allele's presence is associated with the UC subtype; if OR<1, it means this allele's absence is associated with the UC subtype. As an example, rs7668327 is a G/C SNP and the table shows the G allele with OR<1, so that the absence of G (i.e., the presence of C) is associated with the ANCA UC subtype.

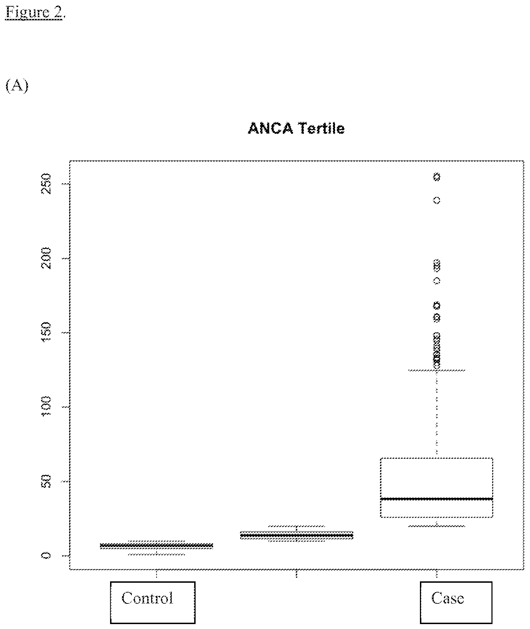

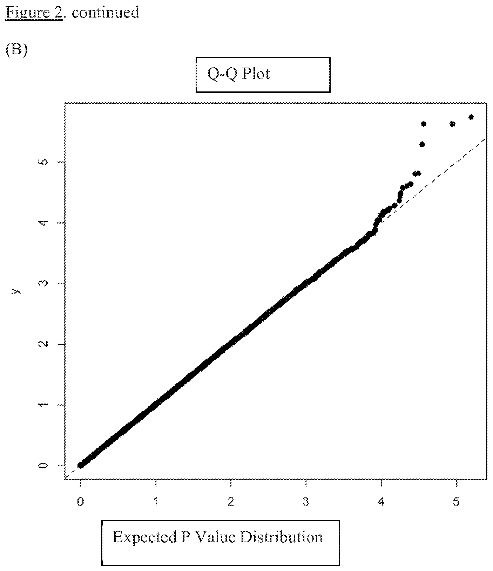

Figure 2 depicts -log(10)p-values of CD ANCA analysis. Figure 2(a) depicts definition of ANCA phenotype. Figure 2(b) depicts QQ plot. The ANCA analysis compared the lowest and highest tertile with the center tertile removed.

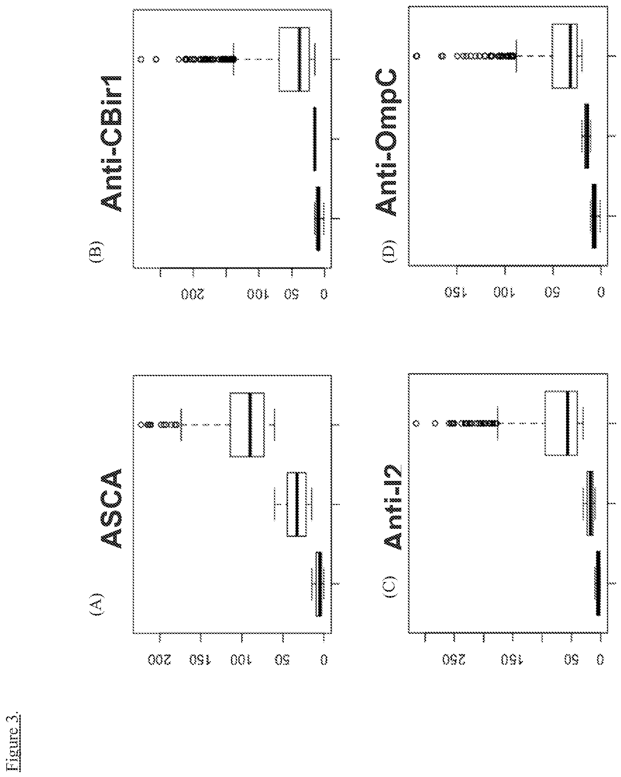

Figure 3 depicts antibody distribution divided into tertiles and scores are then summed. Figure 3(a) depicts ASCA scores. Figure 3(b) depicts anti-Cbir1 scores. Figure 3(c) depicts anti-I2 scores. Figure 3(d) depicts anti-OmpC scores.

### DESCRIPTION OF THE INVENTION

Unless defined otherwise, technical and scientific terms used herein have the same meaning as commonly understood by one of ordinary skill in the art to which this invention belongs. Singleton et al., Dictionary of Microbiology and Molecular Biology 3rd ed., J. Wiley & Sons (New York, NY 2001); March, Advanced Organic Chemistry Reactions, Mechanisms and Structure 5th ed., J. Wiley & Sons (New York, NY 2001); and Sambrook and Russel, Molecular Cloning: A Laboratory Manual 3rd ed., Cold Spring Harbor Laboratory Press (Cold Spring Harbor, NY 2001), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described.

"IBD" as used herein is an abbreviation of inflammatory bowel disease.

"CD" as used herein is an abbreviation of Crohn's Disease.

"SNP" as used herein is an abbreviation of single nucleotide polymorphism.

As used herein, the term "biological sample" means any biological material from which nucleic acid molecules can be prepared. As non-limiting examples, the term material encompasses whole blood, plasma, saliva, cheek swab, or other bodily fluid or tissue that contains nucleic acid.

As disclosed herein, it has been found that response to ANCA has been associated with more aggressive disease behavior in ulcerative colitis (UC) patients, whereas sero-reactivity to ASCA, anti-CBir1, anti-I2, and anti-OmpC have been particularly associated with subtypes of Crohn's disease. There is also a hereditary component to expression of these antibodies. The inventors assessed the genetic contribution to IBD associated serological profiles in UC cases, with 1327 UC cases genotyped with the Illumina CNV370 or OmniExpress beadchips, and were sero-typed for ANCA, ASCA, anti-CBir1, anti-I2, and anti-OmpC by ELISA. The inventors performed regression analyses, adjusted for population stratification using principal components as covariates, testing for an association of UC with antibody response. A Z-score for ASCA, anti-CBir1, anti-I2, and anti-OmpC together was generated by adding the four Z-scores for each individual antibody for each subject. The Z-scores were calculated from within the UC cohort only. Association of UC was assessed with this combined score and with ANCA status alone.

As further disclosed herein, the results demonstrate two genome-wide significant associations with UC and (1) ANCA at chr.4 (rs1919469 p_{logistic} =4.82x10⁻⁸, OR=1.90; rs10001225 p_{logistic}=1.97x10⁻⁷, OR=1.77). An additional three SNPs within this region are also found to be associated with nominal significance (p<10⁻⁵); and (2) at a second region on chr.4 ∼ 37 Mb away, with the combined ASCA, I2, CBir1 and Ompc Z-score (rs2995965 pₗᵢₙₑₐᵣ =1.35x10⁻⁹, β =0.82; rs1863284 pₗᵢₙₑₐᵣ=1.71x10⁻⁷, β=0.85; rs2911920 pₗᵢₙₑₐᵣ=6.29x10⁻⁶, β=0.61). RELL1, a homologue of RELT the TNF receptor that induces epithelial cell apoptosis is located at this locus. In general, these and additional observations disclosed herein support two chromosome 4 loci associated with the phenotypic differences of UC patients, one for UC severity as typified by ANCA level, the other for the expression of antibodies more characteristic of CD.

The present invention provides a method of prognosing inflammatory bowel disease (IBD) in an individual, by obtaining a sample from the individual, assaying the sample to determine the presence of one or more risk variants and risk serological marker according to the method of the invention, and prognosing a severe form of IBD based on the presence of the one or more risk variants and/or risk serological markers. In another embodiment, the present invention provides a method of prognosing a severe form of ulcerative colitis, by determining the presence or absence of one or more of the risk variants (found on Chromosome 4) and ANCA expression, where the presence of the one or more risk variants and a high level of ANCA expression relative to a normal subject are indicative of the severe form of ulcerative colitis.

A method of treating IBD in an individual may be decided by determining the presence of one or more of the risk variants at Chromosome 4.

As disclosed herein, the inventors conducted a genome-wide association study (GWAS) on 1544 CD subjects serotyped for CD-associated antibodies (ASCA, anti-CBir1, anti-I2, and anti-OMPC). Serum antibody expression was measured by ELISA and levels were log transformed prior to analyses. Single nucleotide polymorphism (SNP) data were generated using Illumina technology (∼550K SNPs with MAF>0.05) at Cedars-Sinai Medical Center. Adjustment for population stratification was carried out using two principal components as covariates in the analyses (Eigensoft). The significance of association was tested using logistic regression for antibody positive or negative and linear regression for antibody level after transformation. To overcome multiple testing issues significance was defined to be p < 2e-07.

As further disclosed herein, at the pre-defined level of significance, the inventors observed two significant associations: 1) expression of anti-I2 was significantly associated with three SNPs spanning 90kb of chr. 15 that included the 3 region of human EST BF729345, among other ESTs (rs246336, OR for G allele and anti-I2 positivity, 1.8; p (logistic regression)=8.6e-08); and 2) Expression of anti-OMPC was significantly associated with rs6566234 on chr. 18 (beta coefficient for G allele was -0.28, p (linear regression)=1.4e-07), potentially in LD with CDH19. In addition, 3) anti-Cbir1 positivity was associated with gene AK097193 on chr. 1 (rs1022265 G allele OR for anti-CBir positivity 0.68 p (logistic regression)=7.6 e-07); and 4) ASCA positivity was associated with two SNPs on chr. 3 (rs291528 & rs291523, OR 1.9, p (logistic regression)=5e-07).

The present invention provides a method of prognosing an aggressive form of Crohn's disease in an individual by obtaining a sample from the individual, assaying the sample to determine the presence of one or more risk genetic variants and/or risk serological markers, and prognosing a severe form of Crohn's disease based on the presence of one or more risk genetic variants and/or risk serological markers.

A variety of methods can be used to determine the presence or absence of a variant allele or haplotype. As an example, enzymatic amplification of nucleic acid from an individual may be used to obtain nucleic acid for subsequent analysis. The presence or absence of a variant allele or haplotype may also be determined directly from the individual's nucleic acid without enzymatic amplification.

Analysis of the nucleic acid from an individual, whether amplified or not, may be performed using any of various techniques. Useful techniques include, without limitation, polymerase chain reaction based analysis, sequence analysis and electrophoretic analysis. As used herein, the term "nucleic acid" means a polynucleotide such as a single or double-stranded DNA or RNA molecule including, for example, genomic DNA, cDNA and mRNA. The term nucleic acid encompasses nucleic acid molecules of both natural and synthetic origin as well as molecules of linear, circular or branched configuration representing either the sense or antisense strand, or both, of a native nucleic acid molecule.

The presence or absence of a variant allele or haplotype may involve amplification of an individual's nucleic acid by the polymerase chain reaction. Use of the polymerase chain reaction for the amplification of nucleic acids is well known in the art (see, for example, Mullis et al. (Eds.), The Polymerase Chain Reaction, Birkhauser, Boston, (1994)).

A TaqmanB allelic discrimination assay available from Applied Biosystems may be useful for determining the presence or absence of a variant allele. In a TaqmanB allelic discrimination assay, a specific, fluorescent, dye-labeled probe for each allele is constructed. The probes contain different fluorescent reporter dyes such as FAM and VICTM to differentiate the amplification of each allele. In addition, each probe has a quencher dye at one end which quenches fluorescence by fluorescence resonant energy transfer (FRET). During PCR, each probe anneals specifically to complementary sequences in the nucleic acid from the individual. The 5' nuclease activity of Taq polymerase is used to cleave only probe that hybridize to the allele. Cleavage separates the reporter dye from the quencher dye, resulting in increased fluorescence by the reporter dye. Thus, the fluorescence signal generated by PCR amplification indicates which alleles are present in the sample. Mismatches between a probe and allele reduce the efficiency of both probe hybridization and cleavage by Taq polymerase, resulting in little to no fluorescent signal. Improved specificity in allelic discrimination assays can be achieved by conjugating a DNA minor grove binder (MGB) group to a DNA probe as described, for example, in Kutyavin et al., "3'-minor groove binder-DNA probes increase sequence specificity at PCR extension temperature", Nucleic Acids Research 28:655-661 (2000)). Minor grove binders include, but are not limited to, compounds such as dihydrocyclopyrroloindole tripeptide (DPI,).

Sequence analysis also may also be useful for determining the presence or absence of a variant allele or haplotype.

Restriction fragment length polymorphism (RFLP) analysis may also be useful for determining the presence or absence of a particular allele (Jarcho et al. in Dracopoli et al., Current Protocols in Human Genetics pages 2.7.1-2.7.5, John Wiley & Sons, New York; Innis et al.,(Ed.), PCR Protocols, San Diego: Academic Press, Inc. (1990)). As used herein, restriction fragment length polymorphism analysis is any method for distinguishing genetic polymorphisms using a restriction enzyme, which is an endonuclease that catalyzes the degradation of nucleic acid and recognizes a specific base sequence, generally a palindrome or inverted repeat. One skilled in the art understands that the use of RFLP analysis depends upon an enzyme that can differentiate two alleles at a polymorphic site.

Allele-specific oligonucleotide hybridization may also be used to detect a disease-predisposing allele. Allele-specific oligonucleotide hybridization is based on the use of a labeled oligonucleotide probe having a sequence perfectly complementary, for example, to the sequence encompassing a disease-predisposing allele. Under appropriate conditions, the allele-specific probe hybridizes to a nucleic acid containing the disease-predisposing allele but does not hybridize to the one or more other alleles, which have one or more nucleotide mismatches as compared to the probe. If desired, a second allele-specific oligonucleotide probe that matches an alternate allele also can be used. Similarly, the technique of allele-specific oligonucleotide amplification can be used to selectively amplify, for example, a disease-predisposing allele by using an allele-specific oligonucleotide primer that is perfectly complementary to the nucleotide sequence of the disease-predisposing allele but which has one or more mismatches as compared to other alleles (Mullis et al., supra, (1994)). One skilled in the art understands that the one or more nucleotide mismatches that distinguish between the disease-predisposing allele and one or more other alleles are preferably located in the center of an allele-specific oligonucleotide primer to be used in allele-specific oligonucleotide hybridization. In contrast, an allele-specific oligonucleotide primer to be used in PCR amplification preferably contains the one or more nucleotide mismatches that distinguish between the disease-associated and other alleles at the 3' end of the primer.

A heteroduplex mobility assay (HMA) is another well known assay that may be used to detect a SNP or a haplotype. HMA is useful for detecting the presence of a polymorphic sequence since a DNA duplex carrying a mismatch has reduced mobility in a polyacrylamide gel compared to the mobility of a perfectly base-paired duplex (Delwart et al., Science 262:1257-1261 (1993); White et al., Genomics 12:301-306 (1992)).

The technique of single strand conformational, polymorphism (SSCP) also may be used to detect the presence or absence of a SNP and/or a haplotype (see Hayashi, K., Methods Applic. 1:34-38 (1991)). This technique can be used to detect mutations based on differences in the secondary structure of single-strand DNA that produce an altered electrophoretic mobility upon non-denaturing gel electrophoresis. Polymorphic fragments are detected by comparison of the electrophoretic pattern of the test fragment to corresponding standard fragments containing known alleles.

Denaturing gradient gel electrophoresis (DGGE) also may be used to detect a SNP and/or a haplotype. In DGGE, double-stranded DNA is electrophoresed in a gel containing an increasing concentration of denaturant; double-stranded fragments made up of mismatched alleles have segments that melt more rapidly, causing such fragments to migrate differently as compared to perfectly complementary sequences (Sheffield et al., "Identifying DNA Polymorphisms by Denaturing Gradient Gel Electrophoresis" in Innis et al., supra, 1990).

Other molecular methods useful for determining the presence or absence of a SNP and/or a haplotype are known in the art and useful in the methods of the invention. Other well-known approaches for determining the presence or absence of a SNP and/or a haplotype include automated sequencing and RNAase mismatch techniques (Winter et al., Proc. Natl. Acad. Sci. 82:7575-7579 (1985)). Furthermore, one skilled in the art understands that, where the presence or absence of multiple alleles or haplotype(s) is to be determined, individual alleles can be detected by any combination of molecular methods. See, in general, Birren et al. (Eds.) Genome Analysis: A Laboratory Manual Volume 1 (Analyzing DNA) New York, Cold Spring Harbor Laboratory Press (1997). In addition, one skilled in the art understands that multiple alleles can be detected in individual reactions or in a single reaction (a "multiplex" assay). In view of the above, one skilled in the art realizes that the methods of the present invention for diagnosing or predicting susceptibility to or protection against CD in an individual may be practiced using one or any combination of the well-known assays described above or another art-recognized genetic assay.

One skilled in the art will recognize many methods and materials similar or equivalent to those described herein, which could be used in the practice of the present invention. Indeed, the present invention is in no way limited to the methods and materials described.

### EXAMPLES

The following examples are provided to better illustrate the claimed invention and are not to be interpreted as limiting the scope of the invention. To the extent that specific materials are mentioned, it is merely for purposes of illustration and is not intended to limit the invention. One skilled in the art may develop equivalent means or reactants without the exercise of inventive capacity and without departing from the scope of the invention.

### Example 1

Ulcerative colitis (UC), a subtype of Inflammatory Bowel Disease (IBD), is a chronic inflammatory condition of the gastrointestinal tract with a complex genetic and environmental component. In UC particularly, the environmental factors and the role of bacteria in diseases pathogenesis remains unknown. Response to ANCA has been associated with more aggressive disease behavior in UC patients, whereas sero-reactivity to ASCA, anti-CBir1, anti-I2, and anti-OmpC have been particularly associated with subtypes of Crohn's disease. Furthermore, there is a hereditary component to expression of these antibodies.

The inventors assessed the genetic contribution to IBD associated serological profiles in UC cases. 1327 UC cases were genotyped with the Illumina CNV370 or OmniExpress beadchips, and were sero-typed for ANCA, ASCA, anti-CBir1, anti-I2, and anti-OmpC by ELISA. Regression analyses was performed, adjusted for population stratification using principal components as covariates, testing for an association of UC with antibody response. A Z-score for ASCA, anti-CBir1, anti-I2, and anti-OmpC together was generated by adding the four Z-scores for each individual antibody for each subject. The Z-scores were calculated from within the UC cohort only. Association of UC was assessed with this combined score and with ANCA status alone.

The results demonstrate two genome-wide significant associations with UC and (1) ANCA at chr.4 (rs1919469 p_{logistic} =4.82x10⁻⁸, OR=1.90; rs10001225 p_{logistic}=1.97x10⁻⁷, OR=1.77). An additional three SNPs within this region are also found to be associated with nominal significance (p<10⁻⁵); and (2) at a second region on chr.4 ∼ 37 Mb away, with the combined ASCA, I2, CBir1 and Ompc Z-score (rs2995965 pₗᵢₙₑₐᵣ =1.35x10⁻⁹, β =0.82; rs1863284 p^{linear}=1.71x10⁻⁷, β=0.85; rs2911920 pₗᵢₙₑₐᵣ=6.29x10⁻⁶, β=0.61). RELL1, a homologue of RELT the TNF receptor that induces epithelial cell apoptosis is located at this locus. These observations support that these two loci contribute to the phenotypic difference of UC patients, one for UC severity as typified by ANCA level, the other for the expression of antibodies more characteristic of CD.

### Example 2

It has been reported that CD patients can be characterized by the association of disease phenotypes with the expression of antibodies to microbial antigens. For example CD associated serologies such as ASCA, I2, CBir1 and OMPc are associated with a more aggressive course of disease and an increased chance of surgery. It has also been previously demonstrated the heritable nature of these IBD associated antibodies.

The inventors conducted a genome-wide association study (GWAS) on 1544 CD subjects serotyped for CD-associated antibodies (ASCA, anti-CBir1, anti-I2, and anti-OMPC). Serum antibody expression was measured by ELISA and levels were log transformed prior to analyses. Single nucleotide polymorphism (SNP) data were generated using Illumina technology (∼550K SNPs with MAF>0.05) at Cedars-Sinai Medical Center. Adjustment for population stratification was carried out using two principal components as covariates in the analyses (Eigensoft). The significance of association was tested using logistic regression for antibody positive or negative and linear regression for antibody level after transformation. To overcome multiple testing issues significance was defined to be p < 2e-07.

At the pre-defined level of significance, the inventors observed two significant associations: 1) expression of anti-I2 was significantly associated with 3 SNPs spanning 90kb of chr. 15 that included the 3 region of human EST BF729345, among other ESTs (rs246336, OR for G allele and anti-I2 positivity, 1.8; p (logistic regression)=8.6e-08); and 2) Expression of anti-OMPC was significantly associated with rs6566234 on chr. 18 (beta coefficient for G allele was -0.28, p (linear regression)=1.4e-07), potentially in LD with CDH19. In addition, 3) anti-Cbir1 positivity was associated with gene AK097193 on chr. 1 (rs1022265 G allele OR for anti-CBir positivity 0.68 p (logistic regression)=7.6 e-07); and 4) ASCA positivity was associated with two SNPs on chr. 3 (rs291528 & rs291523, OR 1.9, p (logistic regression)=5e-07).

Examples of rs246336, rs6566234, rs291528, and rs291523 are provided herein as SEQ. ID. NO.: 17, SEQ. ID. NO.: 18, SEQ. ID. NO.: 19, and SEQ. ID. NO.: 20, herein.

These results show that GWAS of serum expression to microbial antibodies may lead to discovery of novel loci affecting CD course and thus targets for therapies for aggressive CD.

### Example 3

Crohn's Disease (CD), a subtype of Inflammatory Bowel Disease (IBD), is a chronic inflammatory condition of the gastrointestinal tract with a complex genetic and environmental component. It has been reported that combinations of genetic and serological markers, including antibodies to anti-Saccharomyces cerevisiae (ASCA), E.Coli outer membrane porinC (OmpC), Pseudomonas fluorescens protein (I2), and anti-flagellin (CBir1), are associated with complications of Crohn's disease. Severe CD is associated with the expression of more than one antibody as well as higher levels of antibody expression.

The inventors identified genes contributing to CD severity by conducting a genome-wide association study (GWAS) of antibody expression in serotyped CD subjects. 1537 CD cases with complete serum antibody profile were genotyped with the Illumina 610quad or OmniExpress beadchips at Cedars-Sinai Medical Center Medical Genetics Institute. Serum antibody for ANCA, ASCA, anti-OmpC, anti-I2, and anti-CBir1 expression was measured by ELISA and log-transformed prior to analyses. 303,147 SNPs with HWE >0.001, MAF>0.02, and GENO >0.02 (genotyping rate=0.9993) were included in analyses. Association for autosomal chromosomes of CD was assessed with Antibody Score using linear regressgion and with ANCA status using logistic regression.

An excess of significance in the tail of the distribution suggests that true positive results are present (below).

**Table 1: SNPs associated with ANCA positive/ negative**

| **CHR** | **SNP** | **Allele** | **OR** | **P** | **GENE(S) IN LD** |
|---|---|---|---|---|---|
| 3 | rs1973780 | A | 1.63 | 3.6x10⁻⁷ | FHIT |
| | (SEQ. ID. NO.: 21) | | | | Fragile histidine triad gene |
| 17 | rs1728171 | A | 0.45 | 3.2x10⁻⁶ | ETV4 |
| | (SEQ. ID. NO.: 22) | | | | ETS variant 4 |
| 6 | rs9449593 | A | 2.1 | 7.7x10⁻⁶ | ME1 |
| | (SEQ. ID. NO.: 23) | | | | Malic enzyme 1 |
| 1 | rs6690359 | A | 0.65 | 9.3x10⁻⁶ | WDR64 |
| | (SEQ. ID. NO.: 24) | | | | WD repeat domain 64 |

**Table 2: SNPs associated with Antibody Score in Crohn's Disease**

| **CHR** | **SNP** | **Allele** | **OR** | **P** | **GENE(S) IN LD** |
|---|---|---|---|---|---|
| 16 | rs1019257 | A | -0.60 | 22x10⁻⁶ | A2BP1 (aka RBFOXl) |
| | (SEQ. ID. NO.: 25) | | | | Ataxin 2 binding protein 1 Trans-golgi network; associated with osteoarthritis and aortic plaque |
| 18 | rs766613 | A | 0.37 | 3.6x10⁻⁶ | CDH2 |
| | (SEQ. ID. NO.: 26) | | | | Cadherin 2 |
| 19 | rs10403164 | A | -0.37 | 9.8x10⁻⁶ | HSPBP1, PPP6R1, BRSK1 |
| | (SEQ. ID. NO.: 27) | | | | Heat shock 70kDa binding protein, co-chaperone 1; Protein phophatase 6 regulatory subunit 1; BR serine/threonine kinase |

These results show genes for antibody expression in CD subjects. These genes are novel with respect to current GWAS results for CD. Because antibody expression is further associated with disease severity, characterization of these genetic associations may add to the list of genetic determinants of CD as well as to the characterization of immune processes that affect CD phenotype.

While the description above refers to particular embodiments of the present invention, it should be readily apparent to people of ordinary skill in the art that a number of modifications may be made without departing from the spirit thereof. The presently disclosed embodiments are, therefore, to be considered in all respects as illustrative and not restrictive.

In some embodiments, the numbers expressing quantities of ingredients, properties such as concentration, reaction conditions, and so forth, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements.

In some embodiments, the terms "a" and "an" and "the" and similar references used in the context of describing a particular embodiment of the invention (especially in the context of certain of the following claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

Preferred embodiments of this invention are described herein, including the best mode known to the inventors for carrying out the invention. Variations on those preferred embodiments will become apparent to those of ordinary skill in the art upon reading the foregoing description. It is contemplated that skilled artisans can employ such variations as appropriate, and the invention can be practiced otherwise than specifically described herein. Accordingly, many embodiments of this invention include all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A method of prognosing inflammatory bowel disease (IBD) in an individual, comprising:
assaying a sample obtained from the individual to determine the presence or absence of one or more risk variants selected from rs10001225(SEQ ID No. 2) rs4694164(SEQ ID No. 3) and rs7668327(SEQ ID No. 4),
assaying the sample to determine the presence or absence of serological marker ANCA; and
prognosing an aggressive form of inflammatory bowel disease in the individual based on the presence of one or more of the risk variants and the presence of serological marker ANCA.

2. The method of claim 1, wherein the aggressive form of inflammatory bowel disease is **characterized by** an aggressive form of ulcerative colitis.

3. The method of claim 1, wherein the presence of serological marker ANCA comprises a high level of serological marker ANCA as compared to a healthy subject.

4. The method of claim 1, wherein the absence of serological marker ANCA is indicative of inflammatory bowel disease with Crohn's like conditions.

## Patentansprüche

1. Verfahren zum Prognostizieren von entzündlicher Darmerkrankung (IBD) bei einem Individuum, umfassend:
Untersuchen einer von dem Individuum erhaltenen Probe zum Bestimmen der Anwesenheit oder Abwesenheit von einer oder mehreren Risikovarianten ausgewählt aus rs10001225 (SEQ ID No. 2), rs4694164 (SEQ ID No. 3) und rs7668327 (SEQ ID No. 4),
Untersuchen der Probe zum Bestimmen der Anwesenheit oder Abwesenheit des serologischen Markers ANCA; und
Prognostizieren einer aggressiven Form von entzündlicher Darmerkrankung bei dem Individuum basierend auf der Anwesenheit von einer oder mehreren von den Risikovarianten und der Anwesenheit des serologischen Markers ANCA.

2. Verfahren nach Anspruch 1, wobei die aggressive Form von entzündlicher Darmerkrankung durch eine aggressive Form von Colitis ulcerosa gekennzeichnet ist.

3. Verfahren nach Anspruch 1, wobei die Anwesenheit des serologischen Markers ANCA ein hohes Maß des serologischen Markers ANCA im Vergleich zu einem gesunden Patienten umfasst.

4. Verfahren nach Anspruch 1, wobei die Abwesenheit des serologischen Markers ANCA auf entzündliche Darmerkrankung mit Zuständen ähnlich Morbus Crohn hinweist.

## Revendications

1. Procédé de pronostic de maladie intestinale inflammatoire chez un individu, comprenant :
l'analyse d'un échantillon obtenu de l'individu pour déterminer la présence ou l'absence d'une ou plusieurs variantes de risque sélectionnées entre : rs10001225(SEQ ID No. 2) rs4694164(SEQ ID No. 3) and rs7668327(SEQ ID No. 4),
l'analyse de l'échantillon pour déterminer la présence ou l'absence du marqueur sérologique ANCA ; et
le pronostic d'une forme agressive de maladie intestinale inflammatoire chez l'individu, en fonction de la présence d'une ou plusieurs des variantes de risque et de la présence du marqueur sérologique ANCA.

2. Procédé selon la revendication 1, dans lequel la forme agressive de maladie intestinale inflammatoire est **caractérisée par** une forme agressive de colite ulcéreuse.

3. Procédé selon la revendication 1, dans lequel la présence du marqueur sérologique ANCA comprend un niveau élevé de marqueur sérologique ANCA par rapport à un sujet sain.

4. Procédé selon la revendication 1, dans lequel l'absence de marqueur sérologique ANCA indique une maladie intestinale inflammatoire avec conditions de type Crohn.


### SEQUENCE LISTING

<110> CEDARS-SINAI MEDICAL CENTER
   HARITUNIANS, Talin
   ROTTER, Jerome I.
   TARGAN, Stephan R.
<120> METHODS OF DIAGNOSING ULCERATIVE COLITIS AND CROHN'S DISEASE
<130> 67789-309WO0
<150> US 61/467,872
   <151> 2011-03-25
<160> 27
<170> PatentIn version 3.5
<210> 1
   <211> 601
   <212> DNA
   <213> Homo sapiens
<400> 1
   agaaaaggca aaagtgggga gaaagcacta aaacgggaga caagttaaaa tttcttttta 60
   attgataggt cacgttctca ctctatttgc ctttaaggga agaaagcaat caagttaata 120
   tgttttcctt cattgtatag tatgtaacta cggacactat tagaggaggg atttgtgtag 180
   cacttaggac attatacttg ataatttcca agggtctttc tagatttaaa agtctgattc 240
   taacgtagta ataaaaataa aggcccaatt ttctctttaa tattgcctga agatattact 300
   stattattgc attaaaatta aacattcaca cattgtttgc actgctaaat aaaattatgt 360
   aatttcttct tctttccttc ctccttcccc catccctctc tatttccctt tccccttcct 420
   tctttcctgg ccttttttcc ttctttcttt gttcccttct ccctccctcc cctttcttcc 480
   tttttctaaa gctggctttg agatccttta ttaaagaata aatctttaaa acttatactt 540
   tattttccct gttgcaggaa aaaatcatgt cctacatatg ttctcaacaa gacactctgt 600
   c 601
<210> 2
   <211> 874
   <212> DNA
   <213> Homo sapiens
<400> 2
   cagactcagt gcattaaatg ccatctagat gttgacaaca catgtttata gctccagcct 60
   ggatttcttc cttgacttac aggctcttac gtcagcttcc tccttaacat ctccatgctg 120
   ataatgtata gacaacacaa acttagcaca ttacaaattg aatgtgaatc tactccttgt 180
   cccaaatttg tacctccatc ttgtctatct gtctgttttt atgagtcaga cattttggat 240
   ttattgatga cccctctctt gcatatcacc cacacattca atctatgagc aaagcttgtc 300
   ggctctacct ttaaaaagta tccagaattt gactatttct caatactttg gtaccagtca 360
   caatcatatg cctcactggg gttattgcaa tagattccta attggtcttt ttacaagtga 420
   ccttgctcca ccctcaaccc tattctcaaa agtgaacatt atgtcacttt tccactcaga 480
   atcctctagt ggcttcccac ytctctcaga gtaaaatgca aagtctgtcc aatgacccta 540
   catgataata tctttctgac cttttttgtt actcctctga tcaggtacag aggtctccct 600
   gctgctcttc aagttaccaa tcaatgtcct gctcagggtt ttgccctcat gacccttctg 660
   gcttagaaga ccctcagcta tgtgtgaggc tcacttcatc accaacttca agcttctact 720
   cgtagttatc tgctaaatga tgatgttttc ctcaactacc ttattttaat tcagaaatgc 780
   cattcccagt ccccacaaca cttatcacca tctgacaaac actagatttt acttgtttat 840
   tttctgtcta cctcactaga gtgtaagctc catg 874
<210> 3
   <211> 1001
   <212> DNA
   <213> Homo sapiens
<400> 3
   ctattatgtt ctctgcctca agtaccagct ctgacaacac atgggcagac tgggaactaa      60
   tgtccccaga agcaatctct tcaactagtg agagatggag ctggtgatag ttcttaagtg     120
   tctccagtgg tgtgaagtct caggtgccca cagtagtgac ccacctatca gcacactctt     180
   aattggcttt cttctcttcc ttgcctcatc ttcttatttc cttactctgc tttcagggat     240
   tgccttccaa atagactctt tctacccaat tctggtttta gagttggttt tggaagaaac     300
   caaaccaaga caatggtcaa gcaagtttat ttctccatga gagccttttg aatagaaacc     360
   tgaatcttgt gagggagtaa gcctcaggaa tatttgaagg aggagaaact aacaaatcca     420
   aagggcttga ggtgggaaca tacttggcaa ttttaaagaa tagcaagaag gccatgtggc     480
   tggagtgggg catgtgaagg yagagagtta ggagatgcct tcagagagtt aggctggggc     540
   tgatccttgt gggcgatgat agagatttag gattttattc tcagtgtcat gtgaaaacat     600
   tggatggttg aggtgggcat taatgtaata tgatttaagt tttaaaatat tctggaaatt     660
   aagtgtagca tattgagcat acatatttat cttttgtgca ataaacatat aaagacaaag     720
   agaactagaa aggaagtcac aacgcggaag aagtattcaa caacattttg aaatctagaa     780
   atatagaaaa gacctaacag atttaacaga ctagagaaag ttgaaaccta agagaagtgg     840
   gggagagaga ggctgttata ggttgaattg tgtcccctaa aagatgatgt gttgtggtcc     900
   taacctctgc tacctcagaa catgacctta tttagaacaa gagtctttgc agatttagtc     960
   atgttaacat gaagtcatta ggtgggcctt aatccaatat a                        1001
<210> 4
   <211> 601
   <212> DNA
   <213> Homo sapiens
<400> 4
   agaaaaggca aaagtgggga gaaagcacta aaacgggaga caagttaaaa tttcttttta 60
   attgataggt cacgttctca ctctatttgc ctttaaggga agaaagcaat caagttaata 120
   tgttttcctt cattgtatag tatgtaacta cggacactat tagaggaggg atttgtgtag 180
   cacttaggac attatacttg ataatttcca agggtctttc tagatttaaa agtctgattc 240
   taacgtagta ataaaaataa aggcccaatt ttctctttaa tattgcctga agatattact 300
   stattattgc attaaaatta aacattcaca cattgtttgc actgctaaat aaaattatgt 360
   aatttcttct tctttccttc ctccttcccc catccctctc tatttccctt tccccttcct 420
   tctttcctgg ccttttttcc ttctttcttt gttcccttct ccctccctcc cctttcttcc 480
   tttttctaaa gctggctttg agatccttta ttaaagaata aatctttaaa acttatactt 540
   tattttccct gttgcaggaa aaaatcatgt cctacatatg ttctcaacaa gacactctgt 600
   c 601
<210> 5
   <211> 874
   <212> DNA
   <213> Homo sapiens
<400> 5
   cagactcagt gcattaaatg ccatctagat gttgacaaca catgtttata gctccagcct 60
   ggatttcttc cttgacttac aggctcttac gtcagcttcc tccttaacat ctccatgctg 120
   ataatgtata gacaacacaa acttagcaca ttacaaattg aatgtgaatc tactccttgt 180
   cccaaatttg tacctccatc ttgtctatct gtctgttttt atgagtcaga cattttggat 240
   ttattgatga cccctctctt gcatatcacc cacacattca atctatgagc aaagcttgtc 300
   ggctctacct ttaaaaagta tccagaattt gactatttct caatactttg gtaccagtca 360
   caatcatatg cctcactggg gttattgcaa tagattccta attggtcttt ttacaagtga 420
   ccttgctcca ccctcaaccc tattctcaaa agtgaacatt atgtcacttt tccactcaga 480
   atcctctagt ggcttcccac ytctctcaga gtaaaatgca aagtctgtcc aatgacccta 540
   catgataata tctttctgac cttttttgtt actcctctga tcaggtacag aggtctccct 600
   gctgctcttc aagttaccaa tcaatgtcct gctcagggtt ttgccctcat gacccttctg 660
   gcttagaaga ccctcagcta tgtgtgaggc tcacttcatc accaacttca agcttctact 720
   cgtagttatc tgctaaatga tgatgttttc ctcaactacc ttattttaat tcagaaatgc 780
   cattcccagt ccccacaaca cttatcacca tctgacaaac actagatttt acttgtttat 840
   tttctgtcta cctcactaga gtgtaagctc catg 874
<210> 6
   <211> 801
   <212> DNA
   <213> Homo sapiens
<400> 6
   ttcttccttt taccttgttt tacaacttgc tcttcttgtc tctttctatt ttttcttttc      60
   cttcccatct taccctccct tcccttccct tcccttccct tgtctacatt cattgcttcc     120
   tgtttcttcc ctcacccgtc ttctctcttt catttttatt ttttatagct ttttatatga     180
   agttgccaga aggaacccat ttgtcttcgc ccctacactt ctaactgttg ctgttcattt     240
   tgaggaggtg gccaaatcat gttgtgaaga acaaaacaaa gtcaactgcc ttcaaacaag     300
   ggtgggtata gcatttgttc catgaagagg ataagaaatc actcaatacc acagatccag     360
   accctgactt atattatagc aaaaggcttg cttaccatat rtgatgttct cttgtcacat     420
   tcagtgattt tcaaaccatt ggtcaccagg tgctgatcta aatattctct ttacttgaga     480
   ctcttgaatg acagccagtc attttcaggg tctctcttcc agctttgttt tctttgtaaa     540
   ttggaaggaa gccctggtga gtgaatgccc agggtgaatg atgacatttt atcttttatc     600
   tttgacaatt tgtagtcttt tgactacaaa caacatggta taatctcagt agggatggcc     660
   aagctcacag catgctgccc caccaaggga gcacttatgc acctttttac caagtgcaat      720
   tcaatcatca gcaagactgg tcctaccttt gtgatgtgcc gtaagtgcat atttctttta     780
   atatgtgtgt ttactgaaat a                                               801
<210> 7
   <211> 605
   <212> DNA
   <213> Homo sapiens
<400> 7
   tttgccaaga gtgtttatgg attacttaat tataaagttt gcatgttgaa atcttaaaga      60
   aacaagcaga ttgacatgac ttgtttaaaa atgcagtatt cattaatgaa ttcaatccat     120
   cagcaatatt tcttcattgt catagatttc ctcagtttta tcagggccat agttgcttgg     180
   aaaaaatatt caccagaaaa aggtttcttt ttaatagtaa ttacttggaa agaatataaa     240
   gttcttcatt tctctcaaat gatagaacac tttccataaa ggttctcacg aagtaatgaa     300
   gactgctaaa cccacatctt ttcttgtttt cttcagctrc attaaatgtc aatctcgtta     360
   gagaagttta ctagatgcgg gtggaaaatc ctatttacaa tttacatatt ccaggtataa     420
   ggataagata agtaagccac aatgactact acagtgtaca tgagaaggct tatctttttc     480
   agaggtcatt actgtaaata gaatcattga aaattgcaag acattaattt gtggaatgaa     540
   tatacagaga gagctgatat attgaagcaa cacacacagg aatattccac tttagcctca     600
   cttcc 605
<210> 8
   <211> 501
   <212> DNA
   <213> Homo sapiens
<400> 8
   tgattacaac agcctgagtc actaagtaca ttttatagat ggtagagtat tgagtttcaa 60
   gaggttcaag tgacttgcct gatgtcacac agtcagtaag gacagggcta agtgtgaaat 120
   tcaggccaac tgtttgcaaa aacaataaca attttcccta taacagccac tgctcgttag 180
   gataaggtgg actaggtctg actaattaag ctgataacca gcgctcttgc taaacaacta 240
   agcctacaag kttattggta ccttcccact ggaatcttat attcatggtt caagaactct 300
   gaatttgtct ttctctcgag aatccattta attcacttca atttatattc atggagtatt 360
   ttactaaata ctaatatttt actaaataaa aagtatatgc ttttattaaa tcatggcttc 420
   ttaatatgta tctattacta ttactattac taaatactaa atattttact aaataaaaag 480
   tatatgcttt tattaaatca t 501
<210> 9
   <211> 601
   <212> DNA
   <213> Homo sapiens
<400> 9
   gggttccttt tccagtttca agtcagatcc acagagcaaa tttcgcattt tcatcaccaa 60
   gaagagagct aaacagatat attagttatt tgcctgtggg aacatacacc ctttcttgtg 120
   ggcagggagc ctgggaaatc ccctcgtatt tcaaaaattc aattgtcaaa aaacaaaagc 180
   caattttgag taatctttga gattttacta taattctgtg tcaaagcaga gtattgagga 240
   aaagtttagt ttgctatatt agcctaagat attgagctta aattaaatat ccagaataat 300
   rgatccagaa aagctattct tattttagag agtttttggc gtattacttt tttttttaaa 360
   gaaatgttgt attcagttgc taatgcccag gataaaaatt agatctatgg ttctcaaact 420
   ccagggggcc tcaaaatttc ctggaaaact tactaaaacg cacagactcc cccaccccga 480
   gtttcagatc cagtaagtct gagatgaggc ctgagaatct gcacttctaa caaattacca 540
   gtgatgccga tactctggtt cagggttcac actttgagaa ccacttgatt aggttgactg 600
   t 601
<210> 10
   <211> 601
   <212> DNA
   <213> Homo sapiens
<400> 10
   ccaagatgtt agacatgcta ctgttattat tatgacttta aattgtgaat tgaattgggt       60
   ggtattgtta gtgaaacagg gaaccttatt ttatattaca ttaatggaat tgtaactcac      120
   ttacttcatg gttatagaag tgtccattaa tagaggctct atttttctgt ctttcttttc      180
   tgtccatcat cagaggcttc atcctttttc tcaccagagc tgcttcactc atggttctat      240
   agagcactgg ggagtctggt tcatcctttg catgtggctt cagggtgttg ctgtgataag      300
   rtaaatagtc tgggaagaat agattataag ctcatatcat tcagctgcct gtgtcctagc      360
   atcctgagtg gatttgtgta tgctttcatt aattttagga atattgctat tttctggctg      420
   tgtaaaagca ttatagtcca ctgcttagct gcatatttac ttattaaatg ttgcctgaga      480
   ctgactcagc aaattctgcc agtatctcat ataaaattct tatgtctcat tcttatgttt      540
   cataagaaat gatcacaatg atcatgtttg gggtttgggc attcctttaa tcttaattta       600
   t                                                                      601
<210> 11
   <211> 8812
   <212> DNA
   <213> Homo sapiens
<400> 11
   tataaactcc ttctgaacag tttcatccac atgcatgttt aagtcaccag ataagtgctg      60
   gttactccta aatataaagc tcaaggtctt acctttctct ttgtttccaa tcatggattc     120
   ttaattgttt cagtttatgg aaccctgtct gctcttcaga atttttgagg accctaaaaa     180
   acttttgctt atgtggatta tatttattaa tgtttaccat attagaaatt aaaactgaga     240
   aaagtttcaa tatttacata ttaatgtatt taaaataata ataaacttgt tacatgttaa     300
   tcttacaaca tattttgtga aaaaaaaact gttttccaaa acaacaacag agaaaagtgt     360
   actaaaaaaa gaccattgtc ttatggtttg aaaatcttaa tatctagctt aatagaaagt     420
   atctagattt tcatatccat tcctgcatgc aatctgtaat agtgagaaaa actggcctca     480
   tatacatatg tacttggaaa gaggaatatt ttaattgttc ctccttttta agattttcag     540
   ataattatgg atattctttt ttgatattac accaaaacta aaaagtagta gttccttaag     600
   ggttagttgc aatgtgaaac ctgaaaccac atcagtgaac ttttcatact ctattataat     660
   aaaatatgct ggtctatttt gaaatttgaa tcgatctttt acccatgcaa gattttgtga     720
   catgcattgg tcatttggaa gacattggtt tactgagtta tgcagatgtt ccgaatgttg     780
   acagattttt cacatcatat gaaaaaataa catttgttaa tatggcgact gatctcacca     840
   gacaagtgtt aaagtatagg taaacctttc caaattctat tttttaattg aaaactcaat     900
   tttcacatga gcaaaacatg caaaggaaac actgcatgtt tcctttgaag tgacaggttc     960
   attttgttca tttttgagaa aatgtcagcc aaagatacaa accatagttt acctgtctgt     1020
   tgttcttcca aataaaaatg gcattccata aaaagtcgcc agctcaaatg gcgattcaaa 1080
   caattttcac aactgttatt ccttgcgata agcattatag actgatatgc agcataaatg 1140
   actcatgaaa tttctagaaa tacaatttta aaagcataaa catatgaaat atgagaagat 1200
   aaaccagtca aaagttgtac ataacttgat attgaaaact atgaactatt attgagagaa 1260
   actaaagatt attttaataa ataaagagag aatctatgat cttaggtcag aagactaagt 1320
   attatttaga ttttaattct ccccaaactg acatataata ttaactcaaa ctcaaaatat 1380
   cagctagagg aaagaggtgg agcaagaagg ccaaatagat ggctctacca attgtttccc 1440
   catgtgggaa taccaagttt aacaactatc tacacaaagg gaacaccttc ataggaacca 1500
   aaaatcgagt gagcactcac agcgcttggt tttgaattcc tatcactgaa agaggcacta 1560
   aagagggtgg gaaagacagt cttgaattcc tgatgccaca cctcctctat tccccagcaa 1620
   caaccacttc tatgcacttg ggagagggat ggggtagcga ttgtgagcct ttccattgaa 1680
   ctcagtgctg ccctgtcaca gtggaaagca gaacggggct gtactcagct tatgcctgca 1740
   cgtggagcaa acattccggg caggccctcg ccagagggga attgccatcc cagcagtcag 1800
   agcttgaatt ccagtaagtg acataaacac aagctggagt gctctggggc cctaagtgaa 1860
   cttagtgggc cgtccaggac ccaaagactg caattcctag gcaagttcta agtgctcttg 1920
   ttcctcaggg aagaacaagc agccataccc atatagcatg ttgtgggctt ttgagactca 1980
   gatgtgcggg cttgagataa caaccagcac attcccagct atgctggcta tgagaagtga 2040
   ctgctaaagc ttgaggaaag cagagggaaa agtaaaggag gggacttcgt cttggacctt 2100
   aggtaccagc tcagccatag taggatagcg caccaagtgg gttcttaggg gttcctgatt 2160
   acaggccttg gtcttagact gaatttctgg acctgtccta agccagaggg gagccgactg 2220
   ccctaaaggg tgagtcccag gcctggcaac atttaataca agttaactga agagcccctt 2280
   ccttgggcct taacggaatg tgagtagtag cctggcagta ctccatgtgg gtctgtggtg 2340
   gtggtggcaa tggggtgaag ctccttggcc tgtgcgaagt ggagggaaga gtgggaagga 2400
   ccgtggcatg tgatttgagt gccagctcag ctgcagtaga ataaaacatc aggtagattt 2460
   ctaagcattt gaatccagtc cttagctccc agaaagcatc tctgtacctc tctggggtct 2520
   tggggaactc agcaccctga aaggaaggaa acgaggatgg ctggcttcat cacctgctga 2580
   ctctagcgtc ctgggacctt gcacaaacat aggcagtaac taggagaaag agagatatgt 2640
   gacctttcag agaattcaaa atagccattt tgaggaaact caaagaaatt caagacaaca 2700
   cagagaagtc atacagaatt ctaccagata aatttaacaa agagattgaa ataattgaaa 2760
   ataatcaaac aaattctgga gttaaaaaat gcaactgata tactgaagaa tgcaccagaa 2820
   tctctaaata gcacaattta tcaaggagaa gaaaagatta gtgacaggcc atttgaaaat 2880
   acagagagga gacaaaagaa aaaaaaatag aaaagaatga aacatgccta caagatctag 2940
   gaaatagctt caaaagggca aatataagag ttatgggcat taaggagaag atagaaaaag 3000
   aaatagaagt agaaattttg tgaaaaggga aaataacaga gaatttccaa aatatagaga 3060
   aagataccaa taagaaggtt acacactacc aagtggattt aacccaaaga tgactacttc 3120
   aaggcattta atcaaactcc caaaggccaa ggataaagaa aagatcctaa aagaggatac 3180
   acacacacac acacacacac acacacacac acacacacac accatataca atgccgcaga 3240
   cagcagactt ttcagcagaa accttacagg ccaggcaaga gtggcatgac atatttaaag 3300
   tgctaaagaa aagaaatttt actctagaat agtatatcca gtgaaaatat ccttaaaaca 3360
   tgaaggagaa ataaagacat tcccagacaa acacaagctg agggatttca tcaacaccag 3420
   acctgtccta caagaaatgc taaagagact actgcaatca gaaagaaaaa gatgttaatt 3480
   aggaagaaaa acatcaccct aaggtataaa actcactggc aatagtaagt atagggaaaa 3540
   acttagacta tcataacact gtaactgtgg cgtgtaaacc actcttaagt agaaagacta 3600
   aaagatgaac taataaaaaa taataactac aaaaactttt taagacagaa acagtacaaa 3660
   gatcactgaa acaataaaat tttttaaatc aggtgatgaa attaaagtgt agagtttttc 3720
   ttagtttact tcttgcatct ttgataaatc gtttacacaa gtactataaa gctgttatca 3780
   gcttaaaata ataggaaata agatagtatt tgcaagcctc atggtaacct tgaatcaaaa 3840
   aaacatatag cagatacaca aaaaataaaa agcaagaaat taaattatac cactgggaaa 3900
   attaccttca gtaaaaggaa gacaggaagg aaagaaagaa agaagaagag accgtaacac 3960
   catcagaaaa tgaacaagaa aattataaga gtaatacctt acttatccgt aataacattg 4020
   aatgtaaata gactaaactc tccaatcaaa agacatagag tggatgaatg gatagaaaag 4080
   tacgacccaa tgatttgttg tctgcaacaa acacaaacta taaagataca aatagactga 4140
   aaacaaaagg atggaaaaag atattccaca tcaatgaaaa ccaaaaaaga ggagtagcta 4200
   tacatacata tatatgtata aatatatatg aaatatatat akagagagaa atccactttt 4260
   tttttgattt gggtcttctt tctttttttc ttagtctgtc taatgattcg ttaattgtgt 4320
   ttatttattc aaaaaaacaa ctttttctgc cattaatttt ttgtattttt ttcatttaaa 4380
   atttatttcc actctgatct ttattatttc tcttctatta gtttatatat atatatatat 4440
   tttatgttta tatatatata tatttgatat atatatatgt ttcaagacaa aaactctaag 4500
   agccaaaaat cactatataa tgataaagga gtcaattcag caacgggata taacaattgt 4560
   agatacatgt gcacccacca ctgtagcacc cagatatagg aagcaaatat taatagagct 4620
   aatgagagag accgatcaca ataaaataat aggtggatac ttcaacgctc tactttgagc 4680
   actggaataa tcttccagac agtaaatcaa taaagaaaca tctgacttaa tctgcactat 4740
   aaaccaaata gacctaatag atatttacag agcatttcat ccaatggcta cagaatatac 4800
   attcttttcc tcaacacgtg gattatactc aacaatagac catatgttaa gtcacaaaac 4860
   aagtcttaaa acattaaaaa atcttgaagt gatattcatc atcttctctg acaacagtgg 4920
   aaacaaaccc tagaaacaaa aacaaatgag caattttggg aactgtacaa acacacagaa 4980
   attaaatgat atgctcctga ataaccagtg ggtcaatgaa gagattaagg aggaaattta 5040
   aaaatgtctt gaaacaaatg acaatggaaa cacaatgtaa caaaactaat gggatacaga 5100
   aaaagtaata ctaagaggga agtttatagc tatgagtgcc tacagcaaaa aacaagaaaa 5160
   acttcaaata aataacctaa caatacatct taaagaacta caaaaacagg agcaaatcaa 5220
   accccaaact agcagaacag aaataataaa gatcagagtg gaaataaatt ttaaatgaaa 5280
   aaatacaaaa aattaatgga acaaaaagtt gggtttttga atacataaat gaaattaacg 5340
   aatctttaac catactaaga aaaaaagatg gaagacccaa atgaataaaa taagagatta 5400
   aaaaaggaga tattacaact gatgctgcag aaatttaaag aatcattagt ggctattatg 5460
   agcaactaca tgccaataaa ttagaaaaat cttaaaaata aataaataaa tttctaggta 5520
   cataaaatct aacaagtttg aacaatgaag aaatccaaaa cctgaacaga ccaataacaa 5580
   acaatgagat caaagccata ataaagtttc ccagcaaaga aaagcccaaa actcaaaggc 5640
   ctccctgttt aattccaaca tttaaagaaa taataccaat cctactttaa ttactctgaa 5700
   aagttgagca ggagggaata cttccaaact cattctatga ggccagtatt actctgatac 5760
   caaaaccaaa cacacacaca cacactcaca cacacacaca gagagagaga gagagaaaac 5820
   tacaggccaa tatctttgtt gaatattgat gcaggatatc ctcaacaaaa tactagcaaa 5880
   ctgaattcaa caatgcattt aaaagatcat tcatgatggc caagtgggtt ttttccagag 5940
   atacaatgat agtttaacat acacaaatca atcgaaatga tacatcatgt caacagaatg 6000
   aaagacaaat actatatgac catttcaaat gatactggga aagcatttga taaaattcaa 6060
   catccctcgt gataaaaccc tcaaaaaact gaacatagaa ggaacatatc tcaattcaat 6120
   aaaagccata ttaaagactc acagctagca tcatactaaa tggggaaaag ctgaaagcct 6180
   tttatctgag aactggaaca taacaaggat gcccgctttt accactgtta ttcgacatag 6240
   tactgcaagt cccagatgga gtatcagtga tgagaaagaa atgaagagcg tccaaattag 6300
   aaagaaagaa gtcaaattat ccctttttta gatgttataa tcttatattt gaagaaaact 6360
   aaagactcaa ccaaaaatga ttagaactaa aaaacaaatt cattaaagtt ccaggataca 6420
   atattaacat acaaaaatta gtagcatttc tatatgctta cagcacaatc aaaaaaaaag 6480
   ctacaaagta atccaattta taatagctac aaataaaata aagtacctag gaattaacca 6540
   aataagtgaa agttatttac aatgaaacta taaaacattg ttgcaaaaga ttgaaatgac 6600
   acaaaaaata gaaagtgttc catgttcacg gattagaaga atcaatattg ttaaaatgtt 6660
   catgctaccc aaagcagtct acagattcaa tacaatcact ttcaaaatac caacaacatt 6720
   ctttacagaa atagaaaata cagctctagg acttatatgg aagaatcaca ttatctgact 6780
   gtaaattata ctacagtggt gtagtaacca aaatcgaatg gtactggcat aaaaacagac 6840
   aaatagacca tagaataaag aatcccaaaa taaactcaga catctacagt gaactccttt 6900
   ttgataaggg ttccaagaac atacattggg gaaagaacag tctcttcatt aaatggtgct 6960
   gggaaaattg gatacctata tgcagaagaa agaaactaca ctcctatctc ttaccatata 7020
   cacaaatcaa atcaaaatac agatgtaaac taagacctca aactattaaa ctacttaaga 7080
   aaacattgga gaaactcttc aggacatggg tttgggcaaa gatttcttga gtaataccct 7140
   acaagcatgg acaaccaaag caaatatgaa caaatggaat aacatctagt taaaatgctt 7200
   ctgcacagaa aaaagcaaat caacaaagtg aagagaatgc acagaatggg agaaaatatt 7260
   tgtgaactac ccatctgaca agcgattagt aaccaggata tatatggagc tcaaacaact 7320
   ccctaggaaa aaagtcagat aatctgtttt aaaaatggga aaaagatgtg aatagatatt 7380
   tgtcaaaaga agacatacaa atgtcaaaca agcttaagaa aatgtgctca acatcgttga 7440
   tcatcatata aatgcaaatc aaaaccacaa cgagatatca tctaacctca gttaaaatga 7500
   tttttatcca aaggataggc aataacaaat gctggcaagg atgtgaagaa aaggaactcc 7560
   tcttggtggg aacgtaaatt agtacaaaca caatggagaa cactgttggt gggaatgtaa 7620
   attagtacag ccactatgaa gaaaagtttg cagattcctc aaaaaactaa aactagcact 7680
   accctatgac ccagcaatcc cacagctagg tatataacca aaaggaagaa aatcagtgtt 7740
   agtgttatct gcatgcccat gtttattgca gcactatcca caatagccaa gatttgaatg 7800
   caacctaagt gtccatcaac agatgcatgg ataagggaaa tgtggtacat atacacaatg 7860
   gaatactatt cagccataaa aaagaatgag atcctgtcat cctgtcattg caataatatg 7920
   gatggaactg gaggtcatta tgttatttaa aataagtcag acagggaaag tcaaacttca 7980
   cgtgttctca cttatttgtg aaaactaaaa attaaaacaa ttgaactcat ggagatggag 8040
   tagaagaagg gatatcagag aataggaagg gtagtcagtg gggttggtgg gaacagagtg 8100
   atggttagtg ggtacaaaat aagtcagagg gaatgaataa gacccgatgt ttgctagcac 8160
   aacagggtga ctatagtcaa aagttatttt aaaataacta agagagtata attgtatttt 8220
   tttaacatag aggataaatg cttgagggaa tggatacccc atttgcccga tgtgattact 8280
   gtgtattgca tgcctgtatc aaaatatctc atgtaacccc aaaatataca caccctacta 8340
   tgtactcaca aaaaattaaa aattatatat atgttcatat atgacacata tatcagctaa 8400
   atttttaagt aaaaaatggc aagctaatcc taaaatttat atggaaattc aaagaaccta 8460
   gaagagcaaa acaactttgc aaaataataa taatattaga taattaatac aacctgactg 8520
   caaggtatat tataaaggaa gcgtaatcaa tgttatgtgg tattgagata atgttaggca 8580
   aatgtatcca tgaaacagga taattttctg gaaaagatct acatacaaat gaacagatta 8640
   ttttctacaa aagtagcaaa attcagttga gaaagtgtgg tatagtaaat tttattcaca 8700
   caatctaaat taagcctcca taaagtttat ttaaacagaa gaaataggtc atagtgcccc 8760
   caaaatttgg gaactactca agattcctgg gattgttgga ccaacatgaa at 8812
<210> 12
   <211> 1127
   <212> DNA
   <213> Homo sapiens
<400> 12
   agaaagggta gaactgaatg aaggtctact caggctagct ttctcttttc tggcagcatc 60
   atcaggcaca ttgaatttca catttttttt tattttttag ttttttgaga cagagccttg 120
   ttctgtcaca caggctggag tgcagtggca tgatcttggc tcactgcagc ctctgcttcc 180
   tgggttcaag aaattctcct gcctcagcct cttgagtagc tgagattaca gtcgcacgcc 240
   atcatgcctg gctaattttt gtatttttaa tagagagggg gtgtcgccat gttggtcagg 300
   ctggacttga actcttgacc tcaggtaata cacctgcctt ggcctcccaa agtgctagga 360
   ttacaggcat gagccaccgt gcccagctga acttcacata tttttaaatg taaaaaacat 420
   ttttaaatgg aagtcagtca tgattttctt cacaaacatt tcatttcact gcaattctag 480
   cttttctccc cattaagaat agagagtgtg ttagtccatt ttgtgtttct ataaaggaat 540
   accagagact gggtaatttg tttttaaaaa agaagtttat ttgtctcatg gctctgcagg 600
   ctgtagaagc atggcatcag catctgcttt gcttcagggg aggcctcagg aagcttttac 660
   tcatggaaga aggtgaaggg aaagcaagtg tgtcacatga agagagaggg tgcgagagag 720
   agagagagag tggggagatg ccacactctt ttatacaaca agatctcagc tgaacttayt 780
   accactcatt accttgagga aagcaccaag ccattcatga aggatccatc cccatggccc 840
   agcacctccc actaggcccc atcaccaaca ctggggaatc acatttcaac atgagatttg 900
   gagtgaacaa atatcccagc catatcaaaa gggttatttt caattgttat tgtgaacgtg 960
   ttctcttctg tcttgtcaaa gctcattaac actgcacact caatctgcag acaaaatggt 1020
   ggacaagatt gttgaatggg tcagtgaata tccgtccaag gctgaggcac ttctgtaggg 1080
   ctcaacaagc tgtggcaatc aacaacagtc caaccgagca acacaca 1127
<210> 13
   <211> 831
   <212> DNA
   <213> Homo sapiens
<400> 13
   tttattatga aacgtcactt tttaaaagat ataatgtttc acatatttta aatcgattta      60
   tttggagaca agtcttttgt cgtaggatca agtatgcgtc taagaggaaa atagacggta     120
   ggtatatgga aagtgtgttt tctgtggctt ttctggttgg cttacttgct ctgtttgctc     180
   taaagactat taggaggaaa rtgataacta agaaaacaga aagtttgttc aaaatggtgt     240
   ggaggaaaaa ggaaggaaag aaaattttta tctgaagaat gagagcccct ttaaattatc     300
   aggccccaaa aggcactgaa tgaaacagca gtcacatcac tcccccttga actaaataat      360
   tatctcttga agccacttgc catgtgggct ctagactaac tgatgccaag tagccatcaa     420
   atgccatatg ccagacacta taacactata tcctgcagtc ccacagtgca tggccaatca     480
   ttaagcaatg ttattctctg aaaaccagtg agaattcctg tcaaagaact ttacatcaga     540
   ccattccttg ttccctttgc ctttagaaac ctgcttacaa caaaagctaa cagagcactc     600
   cccagtgcaa ctgggaagag cgtctcaaca gctgtcttcc ctttggctca agtaaattct     660
   ttaaattata ttttgtgcct cagcctcctc cttttaggtc aacaaggaca tcaactaata     720
   cctccaggtg tgtgcagctt tagtattcac ataaataaga ttttttaaag ccttaatttt     780
   tagcaaccgt cactaatcat taagcccatt cacattttct aaatcgagtg t              831
<210> 14
   <211> 601
   <212> DNA
   <213> Homo sapiens
<400> 14
   tatttatata aataatatac gcttatatat gtatatttga ctaaaaattt aacttaattt      60
   ggaacagcgt tgctactgaa aatgaagttg atatagtgct tttatcccac aggtttgttt     120
   acatattagg ggtttagcaa atagattttg gtgttaaagg aaagggtgcc ctcttcaggc     180
   aatatgtctc atattgctaa agattttgac tagaaaaatg tggacttaaa aaaatactaa     240
   aaactcatag aatcacacca cgaagaggca ttttgtttgg tgttctgtgt ctattcttac     300
   ycagtacaaa gcattttatt tttagaaaac tccaggtaag gatttttaaa tttccatttc     360
   ataatgtgac ttagtttagt tttttttaaa tctctatttc ctttttatct taatatctat      420
   ttcttttcaa catatgagat accttaggct cctttcttgg aagatttaag gtacaactac     480
   agaaatgctt gtcatgggat acctcttttg gcctgtatta acagcagtga tgggaaagca     540
   ttccagaatt cgatttcttt tcaaaaagga aaaacctact aagatctaag atttaatggg     600
   g                                                                     601
<210> 15
   <211> 3900
   <212> DNA
   <213> Homo sapiens
<400> 15
   aaacattgaa tggttaattt tatgtaacat taatggtata tctaatacac agatacaaga 60
   gattgtaatg aacaagagag gtagaatttt taaaaaggct cagaaaagta aattaacaac 120
   cccaaatcac agaatcaatc tgatggataa acattaaata ttagttttat taatggtaaa 180
   acctggattg aggctttgca tcaaaaatar cacattgtcc acatacattg acttccacct 240
   cttctaaaac cacactaaaa tggaaataaa gggatatatt tggaagacat aaatccacaa 300
   ggacaaagag aatgagacga ggtgggaaac cacagcaaat tgtggagggt ggaaagtggt 360
   tggaagagtg gggactgtca tggcagcctt gagagaactg aatcccacac tggctgtggg 420
   aaagccaaga gcaactagtt caaaggtaga ctccctctac cacacacaga ttcaggaatt 480
   gatagaccca gatacttctg caagtcaagg tgaaggtggc ttagaccaga aagattgaac 540
   aaaaatctgt ttaagaagca gttagatctc tagactcctt gtaccccatg ttagtggtga 600
   ttgcccgttt actacacaca ccagcagaag gctggagatt tattctctag catttacatt 660
   gaggatttct tgactgagga accctagata tggattaagt tcaggacacc aaagtgaaga 720
   taggaggaat aaataaaaat tgatatacta tgaatattga taccacccca tgcttcttgt 780
   tgatctattt tcctggaatg ctggtagaac cttatgagat tgtaacctgc ccataaagaa 840
   agaacaagaa ctattgatgt tgacagttcc cccagtgaaa tgctcagcca agtcacctaa 900
   cagtgaagcc catatagcac gcagaacttc tgatcaactt tttgtcccct acccttaaat 960
   acgagatgac agccgaaagt caccacattt tgagcaaacc ctccaatatg aaagacaaag 1020
   gccaaaataa agaaaccaca agaagaaaat aatctatatg aaagagagat ttatgcatgg 1080
   aaaagataat gtgaaaaaaa cacaaaatta atatcttaag aaagatgagg aaaaattgtg 1140
   cttttcttat agttaaatat gagattatat gtatataaga atattattat cttataatta 1200
   aaaataagag aagatttagg gaatagaaaa tagttcatga aaattaaaaa cataatagta 1260
   aaaatgaaaa tttccaaagc atgtttagaa gaagaaaaag cgcaggaagt cttcctaaaa 1320
   gcagaacaaa aaaaattaaa aaataaagag aaaatttttt tttgacagga tcttgctctg 1380
   ttgcccaggc tggagtgcag tggcataaac acagctcact gcagcctcaa cctcttgacc 1440
   tcaagcaatc ctcccacttc agcctcctga gtagctggga gcacagccat ttgccactag 1500
   gctcagctaa tttttaaaaa ttctttgtag agatgggggt cccaccatct tgcccaggct 1560
   ggtctcaaac tcctgggctc aagcgatcct cctgccctag tctcccgaag tgctgggatt 1620
   acaggcatga gccaccacac ccagcctaga aaagtgtttt ataaatgaga aatcaaagga 1680
   gtaatttagg aattacagaa aaggagagca gagaaagtgg agggaaagaa tcatcaaaga 1740
   aataattcaa gaaaaattcc cagatgtaaa tttccaaatt aaaagggccc tctgagtaca 1800
   cagtgaaata catctaagac ccatattaag acacatgata gtgaaatttc agagcattct 1860
   tgccaaatga gggagtgaac ccaagaagat gacatgggat ctgagaaaca ggtgattcat 1920
   aagagaaaaa tgaaaagtat ctctagaacc atgttacagg aagacattga aatgacctct 1980
   gcgcagctga cctatcagtc aatcccaatg gagcaggtca gaaagcacca ggagagacag 2040
   cttcataaag ttcagaatgg tagaacacct aatggatttg aaagcactga gaggagattg 2100
   actcaactgt aggaaaagtt gggaataaat tagcattagg tattagaaat gtaggccgtc 2160
   ttttaaaaag gcaattatat aagttctaga gaaataaatt gtcaggaaag gaaaataagt 2220
   aatatatgat ttagctgcca acagtgtttc cattttacat ggccataaaa tagaaacact 2280
   gaatattgat ctaaccaaac ttacaacata gtgggagggt gagacattgg gaggagtgtt 2340
   tgtgtgtctg tgtctagacg tattttttaa aatgtaaaat caggcctggc actttgattc 2400
   acacctgtaa tcccagcact ttgggaggcc gaggtagggg gaacgctttg agcccaggag 2460
   tttgggacca gcctgggcaa gacagggaga ccccatctct acaaaatttt ttgtttgttt 2520
   gtttttgaga tggagtttca ctcttgttgc ccaggctgga gcacaatggc atgatctcgg 2580
   cctattacaa cctctgcctc ctgggttcaa gcgattctcc tgcctcagcc tccttagtag 2640
   ctgggattac aggcatgcac caccacaccc ggctaatttt gtatttttag tagagacgat 2700
   gttggtcagg ctggtcttga actcctaacc tcaggtgatc tgcccacctc ggcctcccaa 2760
   agtgctggga ttacaggtgt gagtccccac gccccgtcaa aaaaaatttt tttttaatta 2820
   gctgggcatc tggtccccgc tacttgggag gctgaggtgg gaggatcact taaacctggg 2880
   aggttgaggc ttcagtaagg cgtgattgtg ccacttcact ccagcctagg tgatagagtg 2940
   agaccctgtc tcaaaaaata aaaataaaaa gtaaaatcat ctctacagtg ggaaataagt 3000
   gtttaaaagt aaaacaaaca aacaaacaaa caaacaccaa ggcatatcta tataggaatg 3060
   ttcttcagaa ctattgaggg aaatacaaaa gaattggttg aaaagaatga aatgtagttg 3120
   cattattgga aatggtgaag ttgtagagct gccatttttc ttaacaagcc ttttagaatt 3180
   atgtaactct ttaaattatg gacgtgtata actttcattc aaaaatataa aacaaaaaaa 3240
   tctgtattga agaagaggct gtgaggtgaa gtcaaatggg ctgcacatta atttggccca 3300
   cttggtcttt attcaagagt tgaagtctgg ggcaccccaa agtttagagg tacctgagaa 3360
   ggcacagcca gaactgtagg agaaaaacag agagtgtggt gtcttggaag ccaagtgggt 3420
   cgagtgtttc aaggacaaat tgtcagcttt ctcaaatgct tccaagaaga aagtaagaaa 3480
   gttcataaat tggccaggca cagtggctca cgcctgtaat cccagcactt tgggaggtcc 3540
   aggcgggcgg atcacaaggt caggagattg agatcatcct ggccaacgtg gtgaaaccct 3600
   gtctctacta aaaatacaaa aattagctgg gtgtggtggt gcatgcctgt agtctcagct 3660
   actcaggagg ctgaggcagg agaattgctt gaacctggga ggtggaggtt gcgatgagct 3720
   gagatcgggc cactgcgctt cagcctggag ataaagcgag actccatctc aaaaaaaaaa 3780
   caaaaaacaa gaaacaaaaa agaaagttca gaaattacca taggaatgaa gaatacacaa 3840
   atcccaggta ttttcttggg atttccagcc gacagaaaaa aagccattgg cttcctagtt 3900
<210> 16
   <211> 888
   <212> DNA
   <213> Homo sapiens
<400> 16
   gggtgcgctg cagggggtgg gagtgagcga ggaggaattg cggggctagg gtgggttgct 60
   ggaacctggc acgtgtgaga agggtttgcc atcgtctctg atgcatccag gctacagcca 120
   gccgtgagtt cctgctgcgc gccaggcaca tgtcagatgc tgggatacca ggaagtgcac 180
   ccagataggg gcctgaagac tatgtagaaa ctcagagggt caagaacagc cagaaaaaaa 240
   tcttgaaaaa gaaaagcaaa gtaggaagac cagtggaaca taagatagag accagaaaca 300
   aaaatacacg tgtatggaca atttatttca gaggcggggc tgcagagcac aggaaggaac 360
   tatttttggt taatggtgct cggtcaamgg gatatccacg tagggaaaag aaaaaaggga 420
   acttggcccc ctacctccca tcacacacag ctctgaactc cagatcaatt gtatctctaa 480
   gtgcgaaagg tagtgtagca ggaggagccg cagacaaaac tcctcagaca ccggattaaa 540
   gaaggaagag gtttttattc ggccgggagc gtcggcagac tcgcgtctta agagccgagc 600
   tccctgaaaa agaaattcct agccatttta agggcttaca acgctaaggg gtctgtctac 660
   gtgaaagggt catgatagat caagtaagcg tgaggaacgt gactgggggc tacatacatc 720
   agctaacaga acaagaagtt ttacagtgct ttctcatacg atgtctggaa tttacggata 780
   acgccagtag ttttggtcag gggttaatat tattattatt attttaacca ccagggccag 840
   gtggtggcgc caaggtcgtc tagctattta tcttacttct gttttttt 888
<210> 17
   <211> 4552
   <212> DNA
   <213> Homo sapiens
<400> 17
   ggaacagtaa aatacccacg ttaaaagctc tttctctgtg cctccttagc accctctgat      60
   ccttcataga gcccagcaaa aatcaaccat actataagca tccttcacct gccttgcagc     120
   taagctgaag gacaatgagg acaatgatgc cgtcctttct accccatgtc ccaagctccc     180
   agagcagtgc ttggtaaaaa aaaaagggta cttactaaaa tgttggctga ataaaaggaa     240
   gactttgaaa aattaagtta ttttgtaccc ctatagtgtg aattcttttt attgaaattt     300
   aatacactta cagaaaaaaa tgtacatatt ataattgttc atagagctca gtgaattttc     360
   acaaactgaa caaatctgta aaccacaccc agattgagaa agggaacatg acaaacaccc     420
   tgcaagtctc ccttgtgccc tgtgctgggg ggtgccaaca ccaccacctc ctagtctgat     480
   gatgtactag gagattcaca gactcagcct gtagtcatac tcatggctat gttttactac     540
   agcgaaaggc taggaagcaa aatcagcaaa ggaaaaaagc gcatggacaa agtccaggga 600
   aaccaggtgt aagcctccca cagtcctctc ccagcagagt cacataggac acaattcctc 660
   cagcaatcag ttgtgacaac ccatgtgcaa tgttgtatac aagggaagct cattacagac 720
   tcagtgttca gggttctcat tgggagctgc tcacctcatc ctctgcctaa cacatatgaa 780
   aatctgactc ccagaaggaa agcagatgtt gggcataacc cacattgttt gtacgaacag 840
   tttaggcaca gtgagccact cttatcattt agggagtaat gagaaccctc ctgaaatcca 900
   agctttcaga tgccagccaa gggccagcct tgcaaacagg cctgctttct catctctttt 960
   cacacagtcc atctccttat tgaggcttct ttacagcaaa attattatta gtaggaccac 1020
   atgcaaaagg gtgagaccaa cctcaaatat ggacctcagt tcttcctggt aggggtcctg 1080
   gacttaacca gctaaaataa atcccattaa ccataaagtc tatcttagga agccaggtgg 1140
   cttcctcctt aattcctttt tgttgttgtt gttgagatgg agtcttgctc tgtcacccag 1200
   gctggagtgc agtggcgtga tcttggctca ctgcagcctc cacctcctgg gttcaagcga 1260
   ttctcctgcc tcagcctccc gagtagctgg gactacaggt gcacaccacc acacccagct 1320
   aattttttta tttttgtagg gactacaggt gcacaccacc acacccagct aattttttta 1380
   tttttgtatt tttacttttc accatgtcgg tcaggctggt ctcaaactcc tgacctcagg 1440
   tgatctgccc acctcagcct cccaaagtgc cgggattaca ggcatgagcc accgtgccca 1500
   gcccttaatt gctgtctgaa cctttttttt ttttttcaac ctgacctgag gcatcaattc 1560
   aggcacagta taactctgac cagtaagcgg aagctaaccc aaatgctttc cagggctgag 1620
   gggacatcat gagagttgtg gtcatgtaca gaagcacagt cccaggggca caggtggtac 1680
   ccctgtaaac aaagaatttg caatattagg gcacctcaag caggacatcc attaggctgt 1740
   acaggtgaac agacctccta ccaggggaac tcctgcccaa gtgtttccag tcccatctga 1800
   aaagttccca gagtccttgg cttccaaggg actgcctgaa ggcagtgagt cccaaacagt 1860
   tttgcttgtt caggatacaa gttgatccat ctcaygagca tggatgacat ttgtgggaga 1920
   tcttcatgga aagcatagaa gctagaccca ctctgttgtc agtctgaact caaagcactc 1980
   acagcacatg gaaaggcagc actgggaatt ttccttcagc aagaggaggc ttccagggat 2040
   caagcctccc caggtgccag gctttaatta ttttctccaa tattacaaaa acagtcgtgc 2100
   cctgttcagc aatcataaca ttgcattttt tcaaccatct cttactctca gctaggcctt 2160
   tcctctggaa gggttgtatg taagaggcac aaaagcattt ttgtagataa actttttttt 2220
   tttttttttg agatatggtc tggctctgtt gcccaggcta gagtgcggtg gcacgatttt 2280
   ggctcactgc aacctccgcc tcctgggctc aagccatcct cctacctcag cctcttaagt 2340
   agctggaacc aaaggcacat gccatcatgt ccagctaatt ttttgcattt tttgtagaca 2400
   caggatttca ccatgttgcc aggctggtct tgaactcctg aactcaagca atctgcccac 2460
   ctcggcctcc caaagtgctg ggatcatatg catccatcac tgcgcctggc caacattttt 2520
   ttttttaact atcatcctag agttcaagta gataaacgtt tttatgcaac ttaaccagaa 2580
   agacattcag aaattggtaa ttatgtgtgg agggttgaat tatgtcccca caaaaattca 2640
   tgttaaagcc ctaaccattt acgtgtcctt aaaaatgtga gtatatttga agatagggcc 2700
   tgtataggta attaagttaa aatgggatgt ctaggatggg cccttaatcc aacctgagtg 2760
   gtatccttat taaaagagaa gattaggaca cagacaccca gacagaggga ccatacaagg 2820
   acacagtaag aaggcagcca tctgcaagcc aagcagagag gcctcagaag aatctaaagc 2880
   tgtcgacacc ttgtcctgga ctttcagcct ccagaactgc aggaaaataa gtttctattg 2940
   tgtaagtcac tcagtttgtg gtactttgtt atggtatcct tagcaaacta ataaaggatg 3000
   aaaccctgaa tttttaaaaa tttcaaaatg tgtttacata atcttctacc ccttttgtcc     3060
   tgattattca atgagcagac tagaaaagcc taataccttt ctggggacag ctacgtttta     3120
   ataatccacc taccttacta agctgtgtgt gctgggagaa aagtgaggga agtagagtca 3180
   ggctgtcagt ccactgttgc aagctgcaac taatcccaaa agctgaatgc aacaagtcaa 3240
   cagcagtgag agaagcccca gagaaggttt gagtctggtc tgtcaaaagc aggaggagag 3300
   gtcatagtcc ttgcgatgtg cccttcccca gcttggagcg tttggcaaat attagaagtt 3360
   aggaatgcaa ttagcctctg tattagagac aaagagccta tcagcagctt gatcttagat 3420
   ggtcccatta gagaataagc ccttttcagc caggttccag gacgttttca tagtttagcg 3480
   ctccacggag gggtccttaa atctccaacg gccccaacgt ctgagtcctg gtcattgagc 3540
   ctgtgcctgc tttcagttca gcatagctcg tgttgaggct aaaacagctc aaggcagaca 3600
   gtagcaagtt tcagcttaga gtcagggcca ggccattagg atctgagaat ttgagattct 3660
   caacaagcag ctgctttttt tcagcagcag tgaaagcatg ctgcatggcc agacagctgt 3720
   ggtccaagtg acatgcaaag ctgccttttt atcttgttgg tcttagagtc caattgaccc 3780
   actcaaaaaa aatgcatatt agactagaca atcatcagcc tctaagggga gacatttgat 3840
   tttgacaagg gttcattagc aatttaactg tattttcatt ttccaaacag attgcttctc 3900
   tttacatgtt gtctccctct ttcccgaggc tccagtaagc acgaatcaca gcaaaaataa 3960
   tcagttacag cctgttctgg ttccaatatc cgaagaattt aaattccaac ctatccacta 4020
   gtggcaaaaa actagaaagc tgtatcccac taacaccttt catattttat ttttgagtag 4080
   ctttatttga ttagggtgat ccctctagca tttatgaaat taacaagcat aaaacttttt 4140
   ttttaagaga tgagtagctg ggactacagg cgcacgccac tgcgtccagc taaggtatgt 4200
   tttttaagca atgaaatcaa ccagtaaaaa caagttcaaa tatttttcat tcacacactc 4260
   aaggcctgct gagcatccac tgcatgtgtt ttacttgttc cattagaaag gtttattaaa 4320
   aaatggcggg gcacagtggc tcatgactgt aatcccagca cttagggagg ccaagatcgc 4380
   ttgagcccag gagttcaaga ctagtctggg caacatggta aaaccctgtc tctatttaaa 4440
   aaataaacaa atacaagcaa gttttttttt aaaaaatgat atattaaaga aaatcctcct 4500
   aattctctta tgtcttttca tagctgtttc catgttctca tattcccatc ct 4552
<210> 18
   <211> 601
   <212> DNA
   <213> Homo sapiens
<400> 18
   ttgttctaaa aatgtctttt cagtttttca agttgacgaa gcacttaagc cccaggttct 60
   taaaaattat ttgaagtaaa acttaacttt catctattct gtctggagaa tgaattaagg 120
   caatatttcc tttacgtcat gattgaaagt cctatttgca aacttctgct attatgtttt 180
   agtaaaaata ttttatagaa gagttttagg aactctgatt tagttttcaa aggtaactcc 240
   tagaaaagta cttttgaaaa attataattt ttactctata tttcagaagc agtaaggaga 300
   ygctatagat tgatagctct ttatctccac tgacattcag tgatggagct tctttcttca 360
   tttctagcta tgaagattaa gcacctcata aagttgcagt ataaatacct agaaattcat 420
   ctgtgccttt ttctgcatga tattatgttg acaagctggt atcaagcaac ctaacacact 480
   taaagattaa cattaaaact gatataaggt tttacattta aatctttaat ccatcttgag 540
   ttaatttttt gtatatggtg atacataggg gtccagtttc agtcctctgc atatgatagc 600
   c 601
<210> 19
   <211> 1416
   <212> DNA
   <213> Homo sapiens
<400> 19
   aatgcataaa aagtattaga actctccaaa agttcctacg ccatttacac ctccagtatt        60
   gaaaatgata caaagatgaa atgcatagta agttgtaaaa aataatacaa tttaaaatag       120
   taaaaaataa aactaagaaa caaaaccaaa aataaaattt gacatatgaa aaagtatatt       180
   attacaagga tagattgtgg gcaattgcac agagataatc cctaagaact ggcagacttt       240
   cacggtcatt aactatattt tgaagtcttt catcacaatg aatagttgcg tttttctttt       300
   agggcatgac tctcttcaga taatatgttt agattcattt ccaatgtggc actgctcttt       360
   atgaaattct tctatgattc tatgtatact gatatgagca tttcctatta aagctttcta       420
   ccttctgtgc catgcttctg tgttgtttag ggtttacaga aatccatttc acatgccctc       480
   atatacagaa cacaagtttg gcagaaacaa tgctggtgat cgaacagcaa tgccattgtg       540
   aacattacat cttcttatcc tactgtgcac atagtaattt ttgaaacagt cagtaagctt       600
   tactggcttc ttcaggcaaa ttcagcttta attcattaaa acctcctgaa atgtcataag       660
   taggaaggta tatgatgcat ttttaaactg aagttttctt tgttgctgta tgcagtggct       720
   aatccactca tttgactttt ccaccaaatg caaataaaga caataacaag ataatagttt       780
   tgcctaacat gacgtaacta tcctgcatac aactgaaaat gcactaatcc cttccccaga       840
   ctttggcttt ccggatttca acattcggga ttttaatctt ttggggttgt gaatttcaca       900
   attttagaca ttaggaattt tgatctttca ggatttaaac ttttggaatt tcatcattca       960
   ggattatggc atttgaaatt gtgtcttcca ggattatgat ycaaaggcaa ggcactgatg      1020
   taaattcctg tctagagctg aagggctgag aactaggagc atcaaaggca ggagaagata      1080
   aatgtcgcaa ctcaagaagt taggcaaagt taattcaaac ttcttccacc ttcttgctct      1140
   attcaggcct tcaaagggtt agatgatgcc cactcaacat cggagagggc cgtctgcttt      1200
   attagtccac caattcaaat gtttatctct tctagaaaca ccatcacaga cacacccaga      1260
   tattttaacc tggtatttgg ccccctgggg cccagacaat ttggcatata aaattaagta      1320
   tcacagtgcg gatagtatat ttacttaaaa atgttgtgaa tattagcttg ttaattaaaa      1380
   atctatggat tttacttttt aaaaatctgg atttcc                                1416
<210> 20
   <211> 1250
   <212> DNA
   <213> Homo sapiens
<400> 20
   agctttggta atagatccca gattaatttt tagcccaagc aacaggaaat tcctcttagg        60
   ccttagggcc ttaccctgca gaaatatttt tgttggtgcc tatgttagtc aggattgtcc       120
   agagaaacag aatcaatagg agatagatag atagatagat agatagatag atagatagat       180
   agatagatag atgatagata gatgtttgat agaaagacag ataaaagaga ttactataaa       240
   ttattggcty ctgcagttat ggaggcagag aagtcccaca ttctgccacc tgaaagctgg       300
   agacccaggt aagctcttga tgtagttcca aggcctgaga gcaagagagc caatggagag       360
   ccaatcataa tccagaaagg tacaagccct atcactagaa tcctgaatgt tgaaatccca       420
   agagatcaaa atccccaaaa tgtaattcta gaaaaaataa ttttaaaatt atctaaaata       480
   tttatttaca tttttaaagg aggatttatc taagaaacat aaaaacatga atgaatactt       540
   cataggccac tttatacgat aaaataagca ataatataca tatgtttgtg agtataaaca       600
   ctcagatatt ctaacaacag tcacatgggt ataacagtta tgagcagata tactgtgttc       660
   ataaagaggt caaaaaggga aatgtataaa cacatatcac tgtggttggt aattgtgtgc       720
   acctagcttt ataactgagg tcatctgaaa caccatgaca aaccacctaa gtcttttgat       780
   gggaccaatc aaaaccacca tttacaccat cccccaaaga gctaagatct tgagaaattc       840
   tatctttcac aatgtagatg tacagaaagg atatctcttt atttattgag gaagtttcag       900
   catttttatg tatatgcata atgtttaaat acagtccaca ttgtgataat gtacttcatg       960
   gaatcaaatt tgaaaatgca taaaaagtat tagaactctc caaaagttcc tacgccattt      1020
   acacctccag tattgaaaat gatacaaaga tgaaatgcat agtaagttgt aaaaaataat      1080
   acaatttaaa atagtaaaaa ataaaactaa gaaacaaaac caaaaataaa atttgacata      1140
   tgaaaaagta tattattaca aggatagatt gtgggcaatt gcacagagat aatccctaag      1200
   aactggcaga ctttcacggt cattaactat attttgaagt ctttcatcac                 1250
<210> 21
   <211> 1029
   <212> DNA
   <213> Homo sapiens
<400> 21
   caacaaacag actgccttgg agggtgggag gtaggaaaca ggaacagcag gagactgagc      60
   tccataaaat gtaattgtat ttctactgct gcagacatag aaaattatgc ctaaagaagc     120
   cccagagtat aaattggcta ggaacattgt gttctttcta ggcactgttt taagatggtt     180
   agtgacaaat aagaaggttc ataagaaagg tggttgggtc tgaaaacaga tttggagact     240
   aattgatggt gggaatgttt tgcctggaaa aaggaagact tgtagaggga agatagtaac     300
   agagtcactg tttccaaata tctgaagggc tggcaggtgg aaagatatta aatacattct     360
   atgtggctcc agagggcaga tctgtgagta tcggactaca aagtggcaga tatcattagc     420
   tctccataat aacgggtcaa agtgaaaaaa aatgaacact taaagagtgc cttccaaggg     480
   ggtgagcttc ccatccctgg attcactcaa gcacagagca catggccaca attcttcaag     540
   aggatatcta catttggtca gtgcatgggc cacttggatt tgtggtgcct ctctttacac     600
   atttgaccca tttgatcatt accgccattt tcaggtttta caagttctgt aacaaatagt     660
   ggggggtttt ttgtgatcag tattgcaatg tttgggtgac tgcctagtga aacatattgt     720
   tttcattgtt aagttcatgg aaataaaagg taaaaccttt tctttttaac ttagaggaaa     780
   ccacactgta tttatttttt tctcattccc agcctacttc ctgcttttcc tgcagctcag     840
   cattgcatat tgatggtcct tgggctttgc ttgcctcttc acatgtgttt tatttttgcc     900
   tgcaaagtat ttttggtgat ttgctttatt gtgactgtaa agtattkttt aatgagtttg     960
   aatgctgtta ggcatattgc tcattctcta ggcctataca gttgccacga ctgccttcta    1020
   cctaggctt                                                            1029
<210> 22
   <211> 1650
   <212> DNA
   <213> Homo sapiens
<400> 22
   agttctcaag attattaaga cttaggatgt gtcctcaagg agtttgtagt ttgggcaggg      60
   attgagatct tgccctactg tgtggccaga gcataagatg gcaggaaagg gggctggggt     120
   ccagacctaa ttcaaaggaa aggccggggt tctggtatcc tttcatcact tggcatttcc     180
   aggtttcaga ggaagaaaat yaagttaaaa tcctgattcc ccatttctcc ttctggcatc     240
   tctgattggc atcctttaca aggatggaat ttcatgactt gtgtatttgt gtacttgtga     300
   atcagtgacc ctcttgtttt ggactttatg ggagacttgc gtggggagag ggaattgtgc     360
   tcattcacct gggggggttg gggtgatccc cacttgtcaa aggtgtttga accagagcaa     420
   cgccatcttg agtagcagct gggcaaaatg aggctgagac ctgctgggtt gcattcccag     480
   gaggttaggc attcttagtc acaggatgag acagaagatc agcacaagat acaggtccca     540
   aagaccttgc tgataaaaga gaatgcagta aagaagatgg ccaaaaccca cccaaaccaa     600
   gatggccacg aaagtgatct ctggtcgtcc tcactgttca ttatatgtta attataatac     660
   attagcatgc taaaagacac tcctaccatc accatgacag tttacaaata ccatggcacc     720
   ttccggaagt taccctatat agtctaagaa gggtagggac tttcagttct gggaaatctc     780
   tgcccttttc ccaggaaact catgagtaat ccatcccttt ttagcatata ataaagaaat     840
   aactatagcc gggtgtggtg gctcatgcct gtaatcccag cactttggga ggctgaggca     900
   ggaggatcac ttgaggtcag gagttcaaga ccagcctggc caacatgatg aaaacctgtc     960
   tctactaaaa atataaaaaa ttagcctgat gttgtggcgc acagctgtaa tcccaactac    1020
   ttgggaggct gaggcaggag aattgctaga aaccaggagg cggaggttgc agtgagctga    1080
   attgcaccac tgcactccag cttggacaac agagcgagac tccgtctcaa aaaaaaaaaa    1140
   aaaaaaccgg aacaataagc atactcagtc aagcaactca tgccgccgct ctgcctatgc    1200
   agtatccatt ctttattcat ttagtttctt tttttttttt ttgagacgga gttttgcttt    1260
   tgttgctcag gttggagtgc aatggcgcga tcttggctca ctgccacctc cacctcctgg    1320
   gttccagcga ttctcctgcc tcagtctcct gagtagctgg gattacaggc gtgagtcacc    1380
   acgcccggct aattttgtat ttttagtaga gatggggtct ctccatattg atcaggctgg    1440
   tctcgaactc ctgacctaag gtgatccgcc tcctcggcct cccaaagtgc tggaattaca    1500
   ggagtgagcc accacgccca gcccgtttac tttcttaata aacttgcttt cactttactc    1560
   tataaactcg ccccgaattc tttcttctgc aaggtccaag aatcctctct tgggatctgg    1620
   attgggcccc ctttcaggta acatactcat                                      1650
<210> 23
   <211> 1001
   <212> DNA
   <213> Homo sapiens
<400> 23
   taatcgtaag aaaagaaatt cactcaagtt tctcatccat aaatggttac ctgttcaaat      60
   ctgaaacatg atatcaacct gatgaacatc aacctgatga ttatcaaaaa gctcatgatt     120
   ttataaatat atgcccaatc accgtatcat cctgctgagg cttgggagga ggagggaaac     180
   tgataggaac atgcagctca tgatatctct tgtgccagga attatcaaag tcctttgtct     240
   ctgacctaag agtttcatgt cttctgtcaa tatctatgaa attgtggcta acttgttagc     300
   ttgcaagtag agtaaaatct cagatctttc acagtttcat gacagtacaa gattccccag     360
   tgggcctgct ttgtgatgtt ctgagaatct ttcctcaagg cccagacaga gctcacttct     420
   cctattcttt taatgatttt ataaacaact aatttcctat tttgatctaa tttctgctta     480
   aatactaggg tggtttctgt ytcctgtact gaaccctgaa taataaacat gtccactata     540
   ccatatcaga ctttcacttt aaaccttcaa aatgccattc attattaagc actgttctca     600
   acagggaaat atttaccact taatcatcat ttggaaacaa ttttgttttt gctttttttt     660
   aggtaacaat ctagctctgt agcccaggat gcagtgcagt agtgcaatcg cagctcactg     720
   caacctctgc ctcctggatt caagcaattc tcctgcctca gcctcttgag tcagggggga     780
   tcacaggcac acaccaccat gcccggctaa tgtttgtatt tttagtagag atgaagtttc     840
   accatgttgg ccaagctggc ctcgaactcc tgggctcaag tgatctgcct gcctcagcct     900
   cccaaagtgc tgggattagg ggcatgagcc accacaccca gccctggaaa caatattttt     960
   aattgtccca tcactgtcac agaacttgat ctgatgtgtg a                        1001
<210> 24
   <211> 1262
   <212> DNA
   <213> Homo sapiens
<400> 24
   acactaaaac taaaaacatg ttacgattga gacctggagt ctcctacaga taatttaaag 60
   agaaataaga tatctttcta gcaaaagttg agatcattca tgctcagaga tgaagagaca 120
   gaagagagaa tctttcctat ttcttttaaa tgtaatgagt gtattaagat gatactcatt 180
   catgcatctc agtagcacca cttgggaaaa ttacttaata tctttccatt tggtttcttc 240
   atctgtaaat gtaggaatgt ttagcccaga gggccattat aaagatgaaa ggaggtcatt 300
   catttggtta tttaacaaat acttatttat ttattgagtg tctattgtgt tagatactgt 360
   tctgagttct gagttctagg gacaaagcat tgaacaagga ggaccagggc cctgcaatga 420
   gaaagttgac atgctagggg aagagacaga caaacaagca aagtaacaag tacataagaa 480
   aacagtatga taagtatgrt aagaaatagg aaacacaggg ctgggtgtaa tggctcaaac 540
   ctgtaatccc agcactttgg gaggttgagc caggaggatt gcttgaggcc aggagttcaa 600
   aaccagccta ggcaaaatag caagacccca tctctacaaa aacaaagaaa aagaagaaga 660
   aaagaaatag aaaacatagt ggtgcagtgt gattgaatct cacctctgag gaggtgacat 720
   aaaagctgaa gcctgaatga tcaggggaac cagccccgtg aagatctagg aaaaggaaaa 780
   gggcaattta ggcagaaaga agagcaaact gggctgggtg ccgcagttca tgcctgtaat 840
   cccagcactt tgggaggctg aggtgggtgg attatttgag gttaggagtt cgagatcagc 900
   ctggccaaca tggtggaacc ctgtcactac taaaactaca aaaattagcc tggcatggtg 960
   gtaggcacct gtaaccccag ctacttggga ggctgaggca ggagaatcgc ttgaacctgg 1020
   gaggtggagg ttgcagagag ccgagattgc accactgcac tccagcctgg gcaatagagc 1080
   gagactcagt ctcagaaaag aagaagaaga aaaaaaaagc aaactaggca catacatgtt 1140
   tttggttttt ttattgaagc tatattatta tttacagaaa agggaacaga caacatccct 1200
   tcaaactctt tcatataaat tagctttcat cacattaagc agaaagcata taaatcgcca 1260
   tg 1262
<210> 25
   <211> 665
   <212> DNA
   <213> Homo sapiens
<400> 25
   gcaaattatg ttttgatgca ataactcaaa acaatgtgta cctgtccaca aatttaacat      60
   tacctttggg cccaaattga aggacttgtg gcagaatagc tatccttagt gcccagaccc     120
   ctcatttgac acttatctac atacaaccat catgagtttt tcttcaccgt gcctgcatgt     180
   gttcatgtcc ttatgcctca ttatcccacc ttgatcataa actccttcaa ggcaacggcc     240
   atgcctgatc attcatttta tgtccttctg tgttaaccca atgccttgtc catcatcaga     300
   acagggcagg ggctggttga cagatgagta ttcatgccaa caaaaacact cttccygtcg     360
   tacagtttaa agagcccatt tgtctgttat gacatatcac tcgctttggt gtttgttact     420
   ggagtgccca accagtcagc gttgataggt cactggacac attatataat gtgctgaaat     480
   taactgaatt aatattgtca aaatggcttg gcttttaaat gcaggcttct ccttcaaaga     540
   cacgcagcat ggtctggact ctgaaagaca ccacttttgc ataaaaaata cctgcttatt     600
   atgactgtta cagagaacct tattttagtc actgatcctg aaagtcactt taaaaaaaaa     660
   aaaaa                                                                 665
<210> 26
   <211> 2115
   <212> DNA
   <213> Homo sapiens
<400> 26
   tatttattta taaagcacac aattgtttaa aaagatttat tatgcatatg gattatatat      60
   aagtatataa tacactataa agtagagaat ggtgaagcaa tatggaacta tacatttgta     120
   agattttcat gtattacatg aagtagaaca atgttaactg taagtggact gtgaagagtt     180
   aaggacgcat atgttaattt ttagcacgat tacttaaaaa taatactaaa acagtatagc     240
   taaaaagtca ataggaaaat taaaatggaa ttctaaaagg tattcaagtt tataaaaaaa     300
   ggcaataaaa aagaaacaca gaaacaacaa acagagaaaa ctaatataaa acaaataata     360
   aaatggtata ctcacatyca ctcaagcaaa gaattatgtc gcgtagtaat tcattaaata     420
   ctctaatcaa aggcagaggt tgttagaatg aataaaaaga acaaaaccca aacatttgct     480
   atctataagt catatgttta aaatataaaa acagtgtggc tgggaggctt caagttgatc     540
   cccatgattt gcgcctcttc tagtcatgtc cttgcataat cttttcccct tgaatatcag     600
   caggatccag ggctttgttc taaccaatag attatggcaa aggtaaccag atggatgtta     660
   ttacatgtac atagttactg tacataagat tgtaatgtcc atcttgctag gaaactctct     720
   ctccctcctt ggctttgaag acacatgctg ccatgttatg agctgccatg gaaggagtgc     780
   catcagcaca ggatgtaggg cagcctgtgg ctgatggcca tcaagaaatg gaggctcaga     840
   gtcctacagc ctgaaagaac ccgaattctg ccagcagtca cctaagcttg gaatgagatc     900
   cttccccagt ggaacttcta atgagaacac ggcccttgca aacatcctgc ttgcagactt     960
   gcagaggact cagtgaggtg tgtttggata acatagaaag tgtagatgat gcatgggttt    1020
   tattttaaga ggctaaattt tttatagtaa tgttatacgg taaaagataa ctaatccaga    1080
   ttttgatatc tggaagtggg tgtattaatt tgctagggct actgtaacaa aatatcttaa    1140
   actagatggc ttaaacaaga gaaattcatc acctcacagt tctgaaggct ggaagtccaa    1200
   gaccaaggta ctggcaatgc tggtttcttc tgagggttgt gagggagagc tgatttcatg    1260
   cctttcttct agcttctggt ggtttactgg aaatctttgg cctttcttgg cttatatatg    1320
   tatcacctga tctctgcctt cattttctta tggcattctt cctgtgtgca tgtctgcttc    1380
   tgtggataaa tttcttcttt ttatgagagc atagtcatat tgcattagag cccaccataa    1440
   ggaccttaca ttagcttaac ctaatttgca aagactctat ttctaaataa agtcatactt    1500
   acaggtattg ggggttagga ctccaatgtc ttttcagggg aaatgattga aacagtatca    1560
   gtggttacta ccgaaacaat acctaagagt gtggaagtga ctttggaaca aggaagtggg    1620
   aagactagag gattttgagg actgtgataa agaaagatta agttgccttg aactgactta    1680
   gtagaaatct ggaccttgag gaaactctca ttgagggtga cagagaaggt gaggaacata    1740
   ggagtacttg tggaagagag acccatgtta tgcagttgca gaaagcttag tgaaatttct    1800
   cccttagtta tgtttaaaga agaacaccta agtaataaac cttattattt aactaaggag    1860
   atatccaagt aaagtgttga aggtgctgcc tgttcctttt gctgtgtata gtaaaatgtg    1920
   agaggaaaga gataaattga aagaagaaag attaagacaa aagcaaccag gacttgatga 1980
   tttaaaaaat cttcagcctc tccagatggc aaaagattct acaattaatt aatgactttt 2040
   gagcaaagtc agcaacaatc aggagaaatg ctctttcttt aactgtaagg agtacataag 2100
   cttgaaatta ctggg 2115
<210> 27
   <211> 701
   <212> DNA
   <213> Homo sapiens
<400> 27
   gcttggcgct ttgcagtcag gaatgaagcc cacacccagc cacaggcctg ctcctgagac 60
   acagtgcagc ctccatcaac ggcacctggc tcagacactc ccgccagaat ctctgccagc 120
   cctgtccaga agccaggtgt ggctgctcct gcactagcca gaagggaacc tgggcagggg 180
   cccctccacc gccaactctt ggcccgacga ccggcgggct ctgattggta gaggagctag 240
   accaccagcg ggttctgatt ggtgtacaaa acagattccc agagagagtt ctgatttgtg 300
   gagctaatgt cacatgatta ccctacagca aaggatgctg ggaagggaaa tggcggctac 360
   cctagggaaa atttagatgt ctaacctggg aaattcctca aacctagaat tcaggtttgc 420
   caggtggccg aaaaaaggac agatgtccca cacactgttg ctcaggaaag acagggaaga 480
   gtaaatgaca caatggctct rtagatgata aagagcaaca cttttattat tcttccagtg 540
   gctccccaag tcccttaaac ccgtgcccct cctcccgtcc cccatgcacc ctccaaagga 600
   gatgacaaag gcggcagtga aggaggagaa ggaggaagag aaacaccttt attggaagag 660
   ctgtgtggac aagagaacgg ggatgagagt gagagcatgg g 701